(19) Europäisches Patentamt
European Patent Office
Office européen des brevets

(11) EP 3 556 226 B1

(12) # EUROPÄISCHE PATENTSCHRIFT

(45) Veröffentlichungstag und Bekanntmachung des Hinweises auf die Patenterteilung:
**20.01.2021 Patentblatt 2021/03**

(21) Anmeldenummer: **18168401.0**

(22) Anmeldetag: **20.04.2018**

(51) Int Cl.:
***A23L 33/135*** (2016.01) ***A61K 9/20*** (2006.01)
***A61K 31/715*** (2006.01) ***A61K 31/733*** (2006.01)
***A61K 35/741*** (2015.01) ***A61K 35/745*** (2015.01)
***A61K 35/747*** (2015.01) ***A23L 33/22*** (2016.01)
***A61K 45/06*** (2006.01) ***A61K 31/723*** (2006.01)
***A61K 31/729*** (2006.01) ***A61K 31/734*** (2006.01)
***A61P 1/14*** (2006.01)

(54) **KOMPLEX-SYNBIOTIKUM ZUM AUFBAU EINER GESUNDEN DARMFLORA**
COMPLEX SYNBIOTIC FOR ESTABLISHING HEALTHY INTESTINAL FLORA
SYNBIOTIQUES COMPLEXES DESTINÉS AU DÉVELOPPEMENT D'UN MICROBIOTE SAINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.10.2019 Patentblatt 2019/43**

(73) Patentinhaber: **Raab Vitalfood GmbH**
**85296 Rohrbach (DE)**

(72) Erfinder:
- **Raab, Andreas**
  **85296 Rohrbach (DE)**
- **Spieß, Corinna**
  **85296 Rohrbach (DE)**

(74) Vertreter: **Hertin und Partner**
**Rechts- und Patentanwälte PartG mbB**
**Kurfürstendamm 54/55**
**10707 Berlin (DE)**

(56) Entgegenhaltungen:
**EP-A1- 1 696 937**
**DE-T2- 60 020 991**
**US-A1- 2005 220 776**
**US-A1- 2008 219 961**
**US-A1- 2017 100 442**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

## EINLEITUNG

Die Erfindung betrifft eine Stoffzusammensetzung zum Aufbau einer gesundheitsfördernden Darmflora, dadurch gekennzeichnet, dass die Stoffzusammensetzung zwei oder mehr Präbiotika und Probiotika umfassend Lactobacillus plantarum, Lactobacillus acidophilus, Lactobacillus paracasei, Bifidobacterium lactis und Bifidobacterium longum umfasst.

## STAND DER TECHNIK

Billionen von Bakterien besiedeln unseren Darm, darunter auch Milchsäurebakterien, die zu den gesundheitsfördernden Mikroorganismen zählen. Sie zersetzen die vorverdauten Nahrungsbestandteile und sorgen dafür, dass lebenswichtige Nährstoffe vom Körper aufgenommen werden. Zudem tragen sie zu einem funktionierendem Immunsystem bei, indem sie bestimmte Abwehrzellen, insbesondere T-Zellen, aktivieren. Eine gut funktionierende Immunantwort ist enorm wichtig für den Körper, um frühzeitig pathogene Bakterien abzutöten und somit bakterielle Infektionen abzuwehren. Durch diesen Mechanismus können zudem Allergien, Hautprobleme, sowie Asthma gelindert werden. Eine wichtige Aufgabe der Darmflora ist es außerdem, die Darmschleimhaut vor Durchlässigkeit zu schützen. Eine durchlässige Darmschleimhaut kann zum Leaky-Gut-Syndrom führen, welches als Auslöser für schwere Lebensmittelunverträglichkeiten wie Zöliakie zählt. Hierbei lösen sich die sogenannten "tight junctions", die Verbindungen der Darmepithelzellen untereinander. Somit gelangen auch größere Moleküle in den Blutkreislauf und können zum Teil heftige Immunreaktionen auslösen, da der Körper diese als fremd ansieht. Das Leaky-Gut-Syndrom löst im Körper chronische Entzündungsprozesse aus und kann zur ernsthaften Gesundheitsgefahr werden. So wird es inzwischen mit chronischen Krankheiten wie Rheuma, Arteriosklerose, Diabetes und Alzheimer in Verbindung gebracht. Zudem hat die Darmflora Einfluss auf die Psyche und kann Einfluss auf die Entwicklung von Krebs haben. Auch die Bildung von Vitaminen, wie etwa Vitamin B12, ist eine wichtige Fähigkeit der Milchsäurebakterien.

Es wird empfohlen, dass zum Aufbau einer gesundheitsfördernden Darmflora sogenannte Probiotika verabreicht werden. Als Probiotikum bezeichnet man eine Zusammensetzung umfassend lebende bzw. lebensfähige probiotische Mikroorganismen, die bei oraler Aufnahme einen gesundheitsfördernden Einfluss ausüben können. Voraussetzung hierfür ist, dass die entsprechenden Mikroorganismen das saure Magenmilieu überleben und in lebensfähiger Form in den Darm gelangen. Probiotische Zubereitungen enthalten vorwiegend probiotische Bakterien, insbesondere Milchsäurebakterien der Gattungen *Bifidobacterium* und *Lactobacillus.* Darüber hinaus kommen häufig bestimmte Hefen zum Einsatz. Verwendet werden Probiotika in oder als Lebensmitteln, Nahrungsergänzungsmitteln oder (in zumeist höherer Dosierung) in Arzneimitteln.

Als Präbiotika werden für den Wirt, z.B. den Menschen, aber auch Tiere, unverdauliche Nahrungsmittelbestandteile (Ballaststoffe) bezeichnet, die den Wirt günstig beeinflussen sollen, indem sie das Wachstum oder die Aktivität einer oder mehrerer Bakterienarten im Darm gezielt anregen und dadurch einen Gesundheitsnutzen entfalten. Präbiotika gehören neben den Probiotika zu den am häufigsten genutzten Lebensmittelzusätzen. Die für den Menschen unverdaulichen Substanzen (vorwiegend bestimmte Kohlenhydrate) stellen eine selektive Nahrungsgrundlage für Darmbakterienarten wie Lactobazillen und Bifidobakterien dar und können dadurch gezielt die Zusammensetzung der Darmflora beeinflussen. Dadurch können sich Mikroorganismen mit einem gesundheitsfördernden Einfluss für Menschen im Darm akkumulieren. Auch in der Tierzucht werden Präbiotika als Futtermittelzusatzstoffe verwendet.

Probiotika werden heute als Lebensmittelzusatzstoff oder als Nahrungsergänzungsmittel breit vermarktet. In vielen Fällen kann der gewünschte gesundheitliche Nutzen allerdings nicht vollständig realisiert werden. Dies schränkt auch die Akzeptanz der entsprechenden Produkte ein, obwohl der gesundheitliche Nutzen probiotischer Mikroorganismen prinzipiell wissenschaftlich gut belegt ist. Nachteile der am Markt verfügbaren Zubereitungen liegen häufig in der eingeschränkten Stammauswahl, der zu geringen Dosis und der unzureichenden Überlebensfähigkeit der Mikroorganismen im Magen nach oraler Einnahme. Dadurch gelangt eine zu geringe Dosis lebensfähiger Mikroorganismen in den Darm, und der gesundheitliche Nutzen fällt dementsprechend gering aus bzw. unterbleibt vollständig.

Ein prinzipiell Erfolg versprechender Ansatz zur Wirksamkeitssteigerung besteht in der Formulierung sogenannter Synbiotika: Dabei handelt sich um Stoffzusammensetzungen, die als Kombination eines Probiotikums mit einem Präbiotikum die Vorteile beider Zusatzstoffe synergistisch in sich vereinigen sollen. Dabei kommen prinzipiell zwei Mechanismen zum Tragen: Zum einen stellen die Präbiotika Fermentationssubstrate für die probiotischen Stämme dar und verbessern so deren Wachstumsverhalten im Darm. Zum anderen gibt es Belege für die direkte Stabilisierung der probiotischen Stämme durch präbiotische Substanzen im lebensfeindlichen Magenmilieu. In Tierversuchen konnten bei Gabe einer Kombination des Präbiotikums Inulin mit Bifidobakterien (Probiotikum) synergistische antikarzinogene Wirkungen gezeigt werden *(*Carcinogenesis 19, 281 -285, 1998).

DE60020991T2 beschreibt die Herstellung von Tabletten mit lebenden Mikroorganismen, wobei ein schleimbildenes Mittel die Aufnahme im gastrointestinalen Trakt erleichtern soll. Es werden Tabletten beschrieben, die sowohl mehrere Probiotika als auch Inulin und Xanthan enthalten.

US2008/219961A1 beschreibt ein Verfahren zur Stabilisierung von Tabletten mit lebenden probiotischen Bakterien. Zudem wird eine dreilagige Tablette offenbart, welche Lactobacillus gasseri, Bifidobacterium bifidum und Bifidobacterium longum sowie Inulin und Zellulose enthält.

US2005/0220776A1 offenbart eine Kautablette, die als Probiotika die Lactobacillus Stämme paracaei und acidophillus umfasst, wobei die Tablette zudem ca. 93% von Lactitol und Xylitol enthält.

WO2005/056023A1 beschreibt eine Kautablette, die die probiotischen Stämme Bifidobacterium infantis, Bifidobacterium longum und Lactobacillus acidophilus und die Präbiotika Inulin und oligofruktuose umfasst.

US2017/0100442A1 betrifft die Herstellung von Mehrschichttabletten, wobei in den verschiedenen Schichten unterschiedliche Probiotika eingebracht werden. Es werden Tabletten beschrieben, die eine Mischung aus probiotischen Stämmen und den präbiotischen Substanzen Pektin, Hydroxyproloin und Guar umfassen.

Jedoch zeichnen sich die bekannten Synbiotika alle durch gravierende Nachteile bzw. Einschränkungen aus. Zum einen handelt es sich um wenig anwenderfreundliche Formulierung, die eine komplizierte Verabreichung erfordern und somit die Bereitschaft zur regelmäßigen Einnahme verhindern. Weiterhin erfolgt die Verabreichung bekannter Synbiotika in zu geringen Dosen der präbiotischen Zusätze und/oder der probiotischen Stämme. Zudem decken bekannte Synbiotika nur ein sehr eingeschränktes Spektrum von präbiotischen oder probiotischen Komponenten ab und können daher den Nutzen eines Synbiotikums nicht vollständig realisieren.

## ALLGEMEINE BESCHREIBUNG DER ERFINDUNG

Die der Erfindung zu Grunde liegende technische Aufgabe besteht in der Entwicklung einer neuartigen Synbiotikum-Zusammensetzung, die die Nachteile der Synbiotika des Standes der Technik überwindet. Solche Stoffzusammensetzungen sollten ein breites Spektrum von Pro- und Präbiotika abdecken und zudem im Gegensatz zu den Synbiotika des Standes der Technik sicherstellen, dass eine ausreichende Menge an Probiotika die Magenpassage überlebt und so einen positiven Effekt auf die Darmflora ausüben können. Weiterhin sollten Synbiotika in einer einfachen und anwenderfreundlichen Verabreichungsform entwickelt werden.

Erfindungsgemäß wird die vorliegende technische Aufgabe durch den in den Ansprüchen beschriebenen Gegenstand gelöst nämlich durch die Bereitstellung einer Stoffzusammensetzung zum Aufbau einer gesundheitsfördernden Darmflora, die dadurch gekennzeichnet ist, dass die Stoffzusammensetzung zwei oder mehr Präbiotika und Probiotika umfassend Lactobacillus plantarum, Lactobacillus acidophilus, Lactobacillus paracasei, Bifidobacterium lactis und Bifidobacterium longum umfasst.

Es hat sich überraschenderweise gezeigt, dass eine erfindungsgemäße Stoffzusammensetzung, die ein Synbiotikum darstellt, aufgrund der Tatsache, dass mindestens zwei verschiedene Probiotika und zwei verschiedene Präbiotika enthalten sind, eine besonders hohe Überlebensfähigkeit der enthaltenen lebenden Bakterien ermöglicht. Dies wirkt sich insbesondere vorteilhaft auf das Überleben der in der erfindungsgemäßen Stoffzusammensetzung enthaltenen Mikroorganismen aus, deren Überleben innerhalb der erfindungsgemäßen Stoffzusammensetzung verlängert bzw. gefördert wird, sowohl bei Lagerung als auch bei der Magenpassage nach der Einnahme durch ein Individuum.

Die erfindungsgemäße Stoffzusammensetzung hat im Vergleich zu bekannten Synbiotika des Standes der Technik einen verstärkten positiven Effekt auf den Aufbau oder die Stabilisierung einer gesunden Darmflora. Dies ist im Zusammenhang mit z.B. einer oralen Verabreichung vermutlich auf die überraschenderweise stark erhöhte Überlebensrate der in der Stoffzusammensetzung enthaltenen Mikroorganismen nach der Magenpassage zu erklären. So ist es möglich, dass eine besonders hohe Zahl lebender probiotischer Mikroorganismen der Stoffzusammensetzung den Darm erreicht. Zudem sind die in der Stoffzusammensetzung enthaltenen Präbiotika so auf die Probiotika abgestimmt, dass es zu einer besonders starken Wachstumsförderung nach Erreichen des Darms kommt. Zudem führt die spezifische Zusammensetzung des erfindungsgemäßen Synbiotikums nach Erreichen des Darms und unabhängig von der Art der Verabreichung zum Aufbau bzw. zur Stabilisierung einer gesunden Darmflora, wobei die resultierenden gesundheitsfördernden Effekte die Effekte bekannter Synbiotika überraschenderweise überschreiten.

Die erhöhte Stabilität bzw. Überlebensfähigkeit der in der Stoffzusammensetzung enthaltenen lebenden probiotischen Mikroorganismen führt zu einer sehr langen Lagerfähigkeit und Haltbarkeit der Stoffzusammensetzung, da auch nach längeren Lagerzeiträumen über Monate oder Jahre noch eine überraschend hohe Anzahl lebender Mikroorganismen in der Stoffzusammensetzung enthalten ist. Somit sind auch nach langer Lagerdauer die gesundheitsfördernden Effekte der erfindungsgemäßen Stoffzusammensetzung nach der Einnahme durch ein Individuum messbar.

Die erfindungsgemäße Stoffzusammensetzung ist im Vergleich zu bekannten Synbiotika des Standes der Technik in Bio-Qualität und ohne tierische Bestandteile verfügbar und ausführbar. Dies ist für für eine stetig wachsende Gruppe potentieller Konsumenten immer wichtiger und teilweise sogar essentiell für die Kaufentscheidung und Verträglichkeit.

Probiotika im Sinne der Erfindung sind Zusammensetzungen umfassend lebende bzw. lebensfähige probiotische Mikroorganismen, die bei Aufnahme durch einen Wirtorganismus bzw. ein Individuum, z. B. bei oraler Aufnahme, einen gesundheitsfördernden Einfluss ausüben können. Voraussetzung hierfür ist, dass die entsprechenden Mikroorganismen den jeweiligen Wirkort, was im Kontext der vorliegenden Erfindung bevorzugter Weise der Darm ist, in ausreichenden Mengen erreichen. Probiotika werden zu den funktionellen Nahrungsmitteln bzw. Nahrungsergänzungsmitteln gezählt, werden aber auch, zumeist in höheren Dosierungen, in oder als Arzneimittel verwendet. Im Rahmen der vorliegenden Erfindung werden die Begriffe Probiotika und probiotische Mikroorganismen synonym verwendet.

In Probiotika bzw. probiotischen Stoffzusammensetzungen enthalte probiotischen Mikroorganismen umfassen, ohne hierauf beschränkt zu sein, Milchsäurebakterien wie z.B. Milchsäurebakterien der Gattungen *Bifidobacterium* und *Lactobacillus,* Hefen wie z.B. *Saccharomyces boulardii* und *Saccharomyces cerevisiae,* Enterokokken wie z.B. *Enterococcus faecalis* und Enterococcus *faecium,* und coliforme Bakterien wie z.B. *Escherichia coli.*

Probiotische Bifidobakterien umfassen, ohne hierauf limitiert zu sein, Bifidobacterium longum, Bifidobacterium lactis, Bifidobacterium bifidum, Bifidobacterium breve, Bifidobacterium infantis und Bifidobacterium adolescentis.

Probiotische Lactobazillen umfassen z.B. Lactobacillus acidophilus, Lactobacillus bifermentans, Lactobacillus alimentarius, Lactobacillus brevis, Lactobacillus casei, Lactobacillus fermentum, Lactobacillus johnsonii, Lactobacillus oryzae, Lactobacillus delbrueckii subsp. delbrueckii, Lactobacillus casei immunitass/defensis, Lactobacillus reuteri, Lactobacillus rhamnosus, Lactobacillus casei Shirota, Lactobacillus casei CRL431, Lactobacillus panis, Lactobacillus delbrueckii subsp. lactis, Lactobacillus paracasei, Lactobacillus parabuchneri, Lactobacillus plantarum, Lactobacillus paralimentarius (ehemals Lactobacillus kimchii), Lactobacillus salivarius und Lactobacillus rhamnosus.

Präbiotika im Sinne der Erfindung sind für den Wirt, z.B. Menschen und andere Säugetiere, unverdauliche Nahrungsmittelbestandteile, die den Wirt günstig beeinflussen sollen, indem sie das Wachstum oder die Aktivität einer oder mehrerer Bakterienarten im Darm gezielt anregen und dadurch einen Gesundheitsnutzen entfalten. Präbiotika gehören neben den Probiotika zu den am häufigsten genutzten Lebensmittelzusätzen. Präbiotika gehören aufgrund ihrer weitgehenden Unverdaulichkeit für den Menschen zu den Ballaststoffen. Aufgrund ihrer Unverdaulichkeit für den Wirt stellen Präbiotika eine selektive Nahrungsgrundlage für Darmbakterienarten wie z.B. Lactobazillen und Bifidobakterien dar und können dadurch gezielt die Zusammensetzung der Darmflora beeinflussen. Dadurch können sich Mikroorganismen mit einem gesundheitsfördernden Einfluss für Menschen im Darm akkumulieren. Auch in der Tierzucht können Präbiotika als Futtermittelzusatzstoffe verwendet werden.

Die meisten Präbiotika sind oder enthalten Kohlenhydrate, aber auch nicht-Kohlenhydrate wie z.B. Lignin werden als Präbiotika verwendet. Im Sinne der Erfindung umfasst der Begriffe "Präbiotikum" sowohl den nicht-verdaulichen Bestandteil eines Lebensmittels, der eine präbiotische Wirkung aufgrund seiner Unverdaulichkeit für den Wirt und gleichzeitiger Umsetzbarkeit durch probiobiotische Mikroorganismen hat, wie z.B. präbiotische Kohlenhydrate, als auch Lebensmittel an sich, die einen solchen Bestandteil enthalten. Erfindungsgemäße Präbiotika umfassen, ohne hierauf limitiert zu sein, Inulin, Lactulose, Lactitol, Raffinose, Stachyose sowie weitere Fructane und Oligofructose, Chicorée, Schwarzwurzeln, Topinambur, Yacon, Baobab, Inulin, Agaven, Agaveninulin, Glucomannan (Konjak), Alginat, Agar-Agar, Guaran, Guarkernmehl, Xanthan, Pektin, Dextrin, Cellulose, Lignin, Carrageen, Fruktooligosaccharide, Galactooligosaccharide, Apfel, Apfelmehl, Apfelfasern, Apfelpektin, Erbseballaststoff, Haferfaser, Chiasmen oder Chiasamenmehl (Chiamehl) und/oder Bestandteile davon.

Im Sinne der Erfindung bezeichnet der Begriff "Synbiotikum" eine Stoffzusammensetzung, die mindestens ein Präbiotikum und ein Probiotikum umfasst.

Im Rahmen der vorliegenden Erfindung bezeichnet der Begriff "Darmflora" (auch als Intestinalflora, intestinale Mikrobiota oder intestinales Mikrobiom bezeichnet) die Gesamtheit der Mikroorganismen, die den Darm von Menschen oder Tieren besiedeln. Die Darmflora ist an der Verdauung von Nahrungsbestandteile beteiligt und ist essentiell für die Aufnahme von lebenswichtigen Nährstoffen durch den Körper. Neben der Verdauung hat die Darmflora auch Einfluss auf das Immunsystem und eine funktionierende Immunantwort, indem sie bestimmte Abwehrzellen, insbesondere T-Zellen, aktiviert. Zudem wird der Darmflora ein Einfluss auf die Entwicklung oder Unterdrückung von Allergien, Hautproblemen wie Ausschlag und immunvermittelten Hauterkrankungen und Asthma zugeschrieben. Eine weitere wichtige Aufgabe der Darmflora ist, die Darmschleimhaut vor Durchlässigkeit zu schützen. Eine durchlässige Darmschleimhaut kann zum Leaky-Gut-Syndrom führen, welches als Auslöser für schwere Lebensmittelunverträglichkeiten wie Zöliakie zählt. Weiterhin wird der Darmflora ein Einfluss auf chronische Krankheiten wie Rheuma, Arteriosklerose, Diabetes und Alzheimer, sowie auf psychische Erkrankungen und Krebs zugeschrieben. Auch die Bildung von Vitaminen, wie etwa Vitamin B12, ist die Darmflora beteiligt. Veränderungen der Darmflora können in einer Unter- oder Überbesiedelung und in einer Veränderung ihrer Zusammensetzung bestehen. Es können Fehlbesiedelungen entweder im Dick- oder im Dünndarm oder bei beiden gleichzeitig auftreten, die aufgrund der oben beschriebenen Einflüsse der Darmflora mit gesundheitsschädlichen Effekten verbunden sein können.

Probiotische Mikroorganismen haben einen positiven Einfluss auf die Darmflora und erhöhen die gesundheitsfördernde Funktion der intestinalen Mikrobiota. Die mit einer pathologisch veränderten Darmflora assoziierten gesundheitsschädlichen Folgen können durch probiotische Mikroorganismen verhindert oder umgekehrt werden, wenn die Anzahl der probiotischen Mikroorganismen im Darm ansteigt. Eine gesundheitsfördernde Darmflora hat insbesondere einen hohen Anteil an probiotischen Milchsäurebakterien, was u.a. zu einer gesundheitsfördernden Stärkung der Immunantwort und des Immunsystems führt, einer verbesserten Aufnahmen von durch die Darmflora bereitgestellten essentiellen Nahrungsbestandteilen, einer Unterdrückung der Entwicklung von Allergien und immunvermittelten Erkrankungen, wie z.B. Autoimmunerkrankungen und autoinflammatorischen Erkrankungen. Ein hoher Anteil von probiotischen Mikrobakterien und insbesondre probiotischen Milchsäurebakterien in der Darmflora kann zudem zu einer verbesserten Gesundheit im Sinne von einem Zustand von erhöhtem körperlichen und/oder geistigem subjektivem Wohlbefinden führen.

In Ausführungsformen umfasst die Stoffzusammensetzung zum Aufbau einer gesundheitsfördernden Darmflora drei oder mehr Präbiotika. In weiteren Ausführungsformen umfasst die Stoffzusammensetzung zum Aufbau einer gesundheitsfördernden Darmflora vier oder mehr Präbiotika. In anderen Ausführungsformen umfasst die Stoffzusammensetzung zum Aufbau einer gesundheitsfördernden Darmflora fünf oder mehr Präbiotika. In Ausführungsformen umfasst die Stoffzusammensetzung zum Aufbau einer gesundheitsfördernden Darmflora sechs oder mehr Präbiotika.

Offenbart (aber nicht Teil der Erfindung) sind Stoffzusammensetzung zum Aufbau einer gesundheitsfördernden Darmflora umfassend drei oder mehr Probiotika. Offenbart (aber nicht Teil der Erfindung) sind zudem Stoffzusammensetzung zum Aufbau einer gesundheitsfördernden Darmflora umfassend vier oder mehr Probiotika. Die erfindungsgemäßeStoffzusammensetzung zum Aufbau einer gesundheitsfördernden Darmflora umfasst fünf oder mehr Probiotika. In Ausführungsformen umfasst die Stoffzusammensetzung zum Aufbau einer gesundheitsfördernden Darmflora sechs oder mehr Probiotika.

In Ausführungsformen der Erfindung ist die Stoffzusammensetzung dadurch gekennzeichnet, dass sie als Tablette, Kapsel, Pulver oder Granulat vorliegt.

Bevorzugter Weise liegt die erfindungsgemäße Stoffzusammensetzung als Tablette oder Kapsel vor. Diese Verabreichungsformen ermöglicht eine einfache orale Gabe der Stoffzusammensetzung. Zudem ist die Stoffzusammensetzung als Tablette oder Kapsel bei oraler Verabreichung besser gegenüber dem sauren Magenmilieu geschützt, so dass verhindert wird, dass die Stoffzusammensetzung und die darin enthaltenen lebenden probiotischen Mikroorganismen direkt der Magensäure ausgesetzt werden. Hierdurch wird es ermöglicht, dass eine größere Anzahl der Mikroorganismen die Magenpassage nach oraler Verabreichung überlebt und lebend den Darm erreichen können, wo sie ihre gesundheitsfördernde Wirkung entfalten können. Zudem haben diese Verabreichungsformen den Vorteil, dass sie in kleinen Formaten verpackt werden können und anwenderfreundlich sind, da mit der Verabreichung einer Kapsel oder Tablette die gesamte Stoffzusammensetzung aufgenommen werden kann.

Tabletten im Sinne der Erfindung sind einzeldosierte feste Verabreichungsformen von Stoffzusammensetzungen, die unter Pressdruck aus Pulvern oder Granulaten gefertigt werden.

Die erfindungsgemäße Stoffzusammensetzung in Tablettenform hat zudem den Vorteil, dass die Tablettenherstellung verhältnismäßig einfach, kostengünstig und schnelle mit Hilfe von bekannten Tablettenpressen durch Verpressen der einzelnen Bestandteil der erfindungsgemäßen Stoffzusammensetzung möglich ist, was die massenhafte Herstellung in kurzer Zeit ermöglicht. Zur Tablettenpressung sind bevorzugter Weise keine zusätzlichen Materialien notwendig, die über die erfindungsgemäße Stoffzusammensetzung hinausgehen. Die erfindungsgemäße Stoffzusammensetzung in Tablettenform lässt sich gut verpacken und transportieren, ermöglicht eine hohe Stabilität und Überlebensdauer der enthaltenen probiotischen Mikroorganismen, insbesondere aufgrund der räumlichen Nähe zu den verpressten Präbiotika und aufgrund der durch die Tablettenform ermöglichte Abgrenzung von nicht zur erfindungsgemäßen Stoffzusammensetzung gehörenden Stoffen oder Materialien. Zudem ist die Stoffzusammensetzung in Tablettenform einfach einzunehmen und genau dosierbar.

Tabletten haben darüber hinaus den Vorteil, dass die Anwender diese mit einer gesundheitsfördernden Wirkung assoziieren und insbesondere im Vergleich mit Pulvern und Granulaten eine einfache und zeitsparende Aufnahme möglich ist, was zu einer Erhöhung der regelmäßigen Einnahmewilligkeit (Compliance) führt, die für einen verbesserten gesundheitsförderlichen Effekt der Stoffzusammensetzung von Vorteil ist. Ein weiterer Vorteil der Verabreichung der Stoffzusammensetzung in Tablettenform ist, dass die Tablettenhärte im Rahmen des Pressvorgangs eingestellt und beeinflusst werden kann, so dass eine optimale Härte der tablettierten Stoffzusammensetzung, die auf die genaue Zusammensetzung und die Bestandteile der Stoffzusammensetzung abgestimmt ist, erreicht werden kann. Die Tablettenhärte hat einen Einfluss auf die Stabilität der Tablette im Magen und im Darm. Erfindungsgemäße Tabletten können mit einer solchen Härte hergestellt werden, dass Sie erst am Ende der Magenpassage bzw. beim Übertritt in den Darm aufgelöst werden bzw. aufgeweicht sind, so dass im Darm dann eine effiziente Freisetzung der lebenden Bakterien aus der Stoffzusammensetzung erfolgt. Zudem befinden die Mikroorganismen bzw. Bakterien beim Eintritt der aufgeweichten bzw. sich auflösenden Tablette in den Darm in unmittelbarer Umgebung bzw. Nähe zu den Präbiotika der Zusammensetzung, die einen wachstumsfördernden Einfluss auf die Bakterien haben und so zu einer effizienten Vermehrung der probiotischen Bakterien im Darm ermöglichen. Dies ist insbesondere für die Initialbesiedelung des Darmes unter den extremen Bedingungen des mikrobiellen Wettbewerbs in diesem Habitat ein entscheidender Vorteil und erhöht somit die Wahrscheinlichkeit für eine dauerhafte Etablierung der probiotischen Stämme und die damit einhergehenden positiven Wirkungen enorm. Aufgrund der Verabreichung in Tablettenform sind die Probiotika und die Präbiotika bei der Magenpassage zudem in unmittelbarer Umgebung zueinander in der Tablette angeordnet, so dass die lebenden probiotischen Mikroorganismen durch die protektiven, stabilisierenden und überlebensfördernden Effekte der Präbiotika von der sauren Umgebung abgeschirmt werden und Ihr überleben gefördert wird. Auch bezgl. der Überlebensrate der Probiotika unter Lagerbedingungen ist überraschenderweise die galenische Form der Tablette hier von großem Vorteil, da sie die Haltbarkeit (Überlebensrate der Probiotika durch Stabilisierung) über einen langen Zeitraum ermöglicht. Dies führt zur deutlichen Erhöhung der Haltbarkeitsdauer (shelf-life), was ein enormer Wettbewerbsvorteil ist.

In Ausführungsformen der erfindungsgemäßen Stoffzusammensetzung in Tablettenform ist die Tablette beschichtet. Bevorzugt umfasst die erfindungsgemäße Tablette eine Beschichtung auf Ethylcellulose- oder Acrylsäure-Basis.

Tablettenbeschichtungen oder Coatings im Rahmen der vorliegenden Erfindung sind mit spezifischen Vorteilen verbunden. So kann durch eine Beschichtung eine Farbänderung der Tablette ermöglicht werden. Dies ist insbesondere von Vorteil, wenn die erfindungsgemäße Stoffzusammensetzung in Tablettenform ohne Beschichtung eine Farbe hat, die von den meisten Anwendern als wenig appetitanregend angesehen wird, so dass durch eine Änderung der Farbe die Compliance der Anwender erhöht werden kann. Weiterhin kann durch eine Beschichtung die Tablettenstabilität erhöht werden, so dass die erfindungsgemäße Stoffzusammensetzung vor äußeren Einflüssen, wie z.B. dem Magensaft oder auch einer erhöhten Luftfeuchtigkeit in Lagerräumen, geschützt ist und stabil bleibt. Weiterhin kann durch eine Beschichtung eine modifizierte Freisetzung der enthaltenen Probiotika und Präbiotika erzielt werden, so dass sich die Tablette z.B. erst nach der Magenpassage auflöst und die Probiotika und Präbiotika im Darm freigesetzt werden und ihre Wirkung entfalten können. Weiterhin kann durch eine Beschichtung der Geschmack der erfindungsgemäßen Tabletten maskiert werden, so dass ggf. ein unangenehmer Geschmack der Stoffzusammensetzung überdeckt werden kann, um die Anwenderfreundlichkeit zu erhöhen.

Ethylcellulose und/oder Acrylsäure als Basis oder Hauptbestandteile für die Tablettenbeschichtung sind besonders vorteilhaft, da entsprechende Beschichtungen unlöslich in wässrigen Lösungen sind und somit nur langsam aufgelöst werden, wenn die beschichtete Tablette mit wässrigen Flüssigkeiten, wie Magensaft, in Kontakt kommt.

Die Ausführungsform der erfindungsgemäßen Stoffzusammensetzung als Kapsel hat den Vorteil, dass die enthaltenen probiotischen Mikrobakterien durch die Kapsel vor äußeren Einflüssen, wie z.B. dem Magensaft abgeschirmt werden können. Eine Kapsel ist eine feste Verabreichungsform, die eine festgelegte Dosis der erfindungsgemäßen Stoffzusammensetzung umfasst. Erfindungsgemäße Kapseln bestehen aus einer Kapselhülle und einer Füllung. Die Hülle kann z.B. aus Gelatine, Cellulose, Stärke oder Carrageen bestehen und somit aus einem Präbiotikum, das Teil der erfindungsgemäßen Stoffzusammensetzung ist. Sowohl die Hülle als auch das Füllgut können mit Überzügen oder anderen Formulierungstechniken magensaftresistent gemacht werden. Solche magensaftresistenten Kapseln setzen die enthaltene erfindungsgemäße Stoffzusammensetzung und insbesondere die enthaltenen lebenden probiotischen Mikroorganismen erst im Darm und nicht bereits im Magen frei.

In weiteren Ausführungsformen der erfindungsgemäßen Stoffzusammensetzung können die Probiotika oder die probiotischen Mikrobakterien innerhalb der Stoffzusammensetzung in Mikrokapseln vorliegen. Mikrokapseln sind feste Partikel oder Flüssigkeitströpfchen, die mit einem Gelatinemantel oder anderen Substanzen, z.B. arabischem Gummi oder anderen Polymeren wie z. B. Stärke, Cellulose oder Carrageen überzogen werden. Bevorzugt bestehen die Mikrokapseln aus einem oder mehreren Präbiotika. Sie haben eine Größenordnung vom Nanometerbereich bis zu wenigen Millimeter. Mikroverkapselte Substanzen können als rieselfähige Pulver vorliegen und können im Rahmen der vorliegenden Erfindung so in die erfindungsgemäße Stoffzusammensetzung eingebracht werden und z.B. zu Tabletten verpresst oder in Kapseln verpackt werden. Die Mikrokapseln bieten den probiotischen Mikroorganismen einen zusätzlichen Schutz gegenüber dem sauren Magenmilieu und können zu einer erhöhten Anzahl an lebenden probiotischen Mikroorganismen der erfindungsgemäßen Stoffzusammensetzung führen, die den Darm erreichen. Die Vorteile von Mikroverkapselung sind, dass Flüssigkeiten sich in trockene Pulver überführen lassen und ohne Schwierigkeiten zu Feststoffzubereitungen verarbeitet werden können. Unangenehmer Geruch oder Geschmack kann verdeckt werden, flüchtige Stoffe können fixiert werden. Zudem bietet das Hüllmaterial der Mikrokapseln Schutz vor äußeren Einflüssen (Licht, Luft, Feuchtigkeit).

Ausführungsformen der erfindungsgemäßen Stoffzusammensetzung, die als Pulver oder Granulat vorliegen, haben den Vorteil, dass sie sich mit Getränken wie z.B. Wasser vermischen lassen und anschließend getrunken werden können.

Die im folgenden genannten Ausführungsformen der Erfindung und assoziierten Vorteil beziehen sich insbesondere auch auf die Ausführungsformen der Erfindung in Tabletten- und Kapselform.

Es ist bevorzugt, dass die erfindungsgemäße Stoffzusammensetzung in fester Form vorliegt. In Ausführungsformen hat die Stoffzusammensetzung ein Gewicht von 100 - 1000 mg, bevorzugt 200 - 800 mg, besonders bevorzugt etwa 400 mg. Stoffzusammensetzungen von 100 - 1000 mg lassen sich gut in Kapsel- und Tablettenform produzieren. Dabei haben Ausführungsformen mit einem Gewicht von weniger als 1000 mg den Vorteil, dass sie sich leicht oral einnehmen lassen. Insbesondere hat sich gezeigt, dass Stoffzusammensetzungen in Tablettenform, die etwa bis zu 400 mg wiegen, für Anwender besonders angenehm einzunehmen sind. Gleichzeitig ist es überraschenderweise möglich, in erfindungsgemäßen Stoffzusammensetzung mit ca. 100 mg Gewicht eine so große Anzahl lebender probiotischer Mikroorganismen einzuschließen, das auch nach oraler Einnahme und Magenpassage noch so viele lebende probiotische Mikroorganismen den Darm erreichen, dass ein gesundheitsfördernder Effekt erzielt werden kann.

In Ausführungsformen der Erfindung hat die Stoffzusammensetzung ein Gewicht im Bereich von 10 - 4000 mg, 20 - 3600 mg, 30 - 3200 mg, 40 - 3000 mg, 50 - 2800 mg, 60 - 2600 mg, 70 - 2400 mg, 80-2200 mg, 900-2100 mg, 100-2000 mg, 110 - 1900 mg, 120 - 1800 mg, 130 - 1700 mg, 140 - 1600 mg, 150 - 1500 mg, 160 - 1400 mg, 170 - 1300 mg, 180 - 1200 mg, 190 - 1100 mg, 200 - 1000 mg, 210 - 950 mg, 220 - 900 mg, 230 - 850 mg, 240 - 800 mg, 250 - 750 mg, 260 - 740 mg, 270 - 730 mg, 280 - 720 mg, 290 - 710 mg, 300 - 700 mg, 310 - 690 mg, 320 - 680 mg, 330 - 670 mg, 340 - 660 mg, 350 - 650 mg, 360 - 640 mg, 370 - 630 mg, 380 - 620 mg, 390 - 610 mg, 400 - 600 mg, 410 - 590 mg, 420 - 580 mg, 430 - 570 mg, 440 - 560 mg, 450 - 550 mg, 460 - 540 mg, 470 - 530 mg, 480 - 520 mg, 490 - 510 mg oder ca. 500 mg.

In bevorzugten Ausführungsformen besteht die erfindungsgemäße Stoffzusammensetzung zu 40-99,99 Gew.-%, bevorzugt 60-99,8 Gew.-%, besonders bevorzugt 75-99,7 Gew.-% der Zusammensetzung aus Präbiotika. Bevorzugt besteht die erfindungsgemäße Stoffzusammensetzung zu 75-99,7 Gew.-% aus Präbiotika.

Es war ein überraschender Vorteil, dass die Stoffzusammensetzung zu einem so großen Anteil Präbiotika enthalten kann. Insbesondere war nicht zu erwarten, dass entsprechende Zusammensetzungen sich zu stabilen Tabletten verpressen lassen. Der hohe Anteil an Präbiotika hat den Vorteil, dass die Präbiotika einen stabilisierenden Effekt auf die in der Stoffzusammensetzung enthaltenen lebenden probiotischen Mikroorganismen haben, der überraschenderweise umso höher ist, je größer der Anteil der Präbiotika ist.

Durch den hohen Anteil der Präbiotika wird das Überleben der Probiotika im Magen gefördert. Zudem wird das anschließende Wachstum und die Vermehrung der probiotischen Mikroorganismen im Darm durch den hohen Anteil der Präbiotika an der Stoffzusammensetzung weiter gefördert. Dieser Effekt ist insbesondere bei Vorliegen der erfindungsgemäßen Stoffzusammensetzung als Tablette zu beobachten, da hier die unmittelbare Nähe und die Einbettung der probiotischen Mikroorganismen in die Präbiotika gegeben ist. Dadurch, dass der überwiegende Anteil von z.B. 90 % oder sogar 99,99 % der Stoffzusammensetzung aus Präbiotika besteht, sind die Mikroorganismen praktisch komplett von diesen umgeben und können besonders effizient stabilisiert werden.

Zudem ist es in Ausführungsformen der Erfindung möglich, dass die erfindungsgemäße Stoffzusammensetzung ausschließlich aus Probiotika und Präbiotika besteht, ohne die Verwendung weiterer Hilfsstoffe.

In Ausführungsformen ist der Anteil der Präbiotika an der erfindungsgemäßen Stoffzusammensetzung 20 - 99,99 Gew.-%, 22 - 99,8 %, 24 - 99,7 %, 26 - 99,5 %, 28 - 99 %, 22 - 98,5 %, 24 - 98 %, 26 - 97,5 %, 28 - 97 %, 30 - 96,5 %, 32 - 96 %, 34 - 96 %, 36 - 95,5 %, 38 - 95 %, 40 - 94,5 %, 42 - 94 %, 44 - 93,5 %, 45 - 93 %, 46 - 92,5 %, 47 - 92 %, 48 - 91,5 %, 48 - 91 %, 49 - 90,5 %, 50 - 90 %, 51 - 89 %, 52 - 88 %, 53 - 87 %, 54 - 86 %, 55 - 85 %, 56 - 84 %, 57 - 83 %, 58 - 82 %, 59 - 81 %, 60 - 80 %, 61 - 79 %, 62 - 78 %, 63 - 77 %, 64 - 76 %, 65 - 75 %, 66 - 74 %, 67 - 73 %, 68 - 72 %, 69 - 71 % oder ca. 70 %.

Gemäß weiterer Ausführungsformen, enthält die erfindungsgemäße Stoffzusammensetzung pro Gramm $1x10^8$- $1x10^{17}$, bevorzugt $1x10^{10}$- $1x10^{14}$, besonders bevorzugt $1x10^{11}$ - $1x10^{13}$ Kolonie-bildende Einheiten (KBE) der probiotische Mikroorganismen.

Die Anzahl an KBE ist ein Maß für die in der Stoffzusammensetzung enthaltenen lebenden probiotischen Mikroorganismen. Mikroorganismen können unter anderem durch einen *Surfaceviable Count* oder einen Plaque-Assay quantifiziert werden. Dabei wird meistens eine Probe des Materials, dessen Mikroorganismengehalt bestimmt werden soll, auf der Oberfläche eines Kulturmedium-Gels oder im Kulturmedium-Gel möglichst gleichmäßig verteilt, so dass im Idealfall alle Individuen der Mikroorganismen einzeln und weit genug voneinander entfernt zu liegen kommen und bei geeigneten Kulturbedingungen durch Wachstum und Vermehrung jeweils eine mit dem Auge sichtbare Kolonie bilden. Die Anzahl der Kolonien ist unter diesen Idealbedingungen gleich der Anzahl der in der Probe enthaltenen Individuen. Tatsächlich aber können unter realen Bedingungen einige Individuen so dicht beieinander liegen, dass sie nur eine einzige gemeinsame Kolonie bilden, zudem haften oft einige Individuen so fest aneinander, dass sie nicht getrennt werden und sich ebenfalls nur eine Kolonie daraus entwickelt. Unter realen, nicht idealen Bedingungen ist also die Anzahl der Kolonien geringer als die Anzahl der in der Probe enthaltenen Individuen. Deshalb wurde der Begriff Kolonie-bildende Einheit (KBE) eingeführt, die sowohl einzelnen Individuen als auch mehreren aneinander haftenden oder dicht zusammen liegenden Individuen entspricht.

In Ausführungsformen der erfindungsgemäßen Stoffzusammensetzung überleben mindestens $1x10^6$ KBE der in der Stoffzusammensetzung pro Gramm enthaltenen probiotischen Mikroorganismen die Magenpassage. Das heißt, das von den ursprünglich in der Stoffzusammensetzung pro Gramm enthaltenen probiotischen Mikroorganismen noch mindestens$1x10^6$ KBE vorhanden sind, wenn die Stoffzusammensetzung nach oraler Verabreichung vom Magen in den Darm überführt wird. Die Magenpassage der Stoffzusammensetzung kann im Labor simuliert werden, indem die erfindungsgemäße Stoffzusammensetzung Bedingungen ausgesetzt wird, die dem Magenmilieu entsprechen. Entsprechende Bedingungen sind dem Fachmann bekannt und können u.a. die Magenverweildauer, den Magensaft und dessen Zusammensetzung, sowie die Peristaltik und Umgebungstemperatur simulieren.

Stoffzusammensetzung die pro Gramm mindestens $1x10^8$ KBE der probiotischen Mikroorganismen enthalten sind besonders vorteilhaft, da sich gezeigt hat, dass es nach der Magenpassage zu einer Abnahme der KBE Zahl der in der erfindungsgemäßen Stoffzusammensetzung enthaltenen probiotischen Mikroorganismen um durchschnittlich ca. zwei Zehnerpotenzen kommt. Die genaue Abnahme hängt, wie dem Fachmann bekannt ist, von den Bedingungen der Magenpassage (z. B. Dauer, Magensaftzusammensetzung, Peristaltik) und der spezifischen Zusammensetzung der erfindungsgemäßen Stoffzusammensetzung sowie der Verabreichungsform bzw. Formulierung (z.B. Tablette, Kapsel, Pulver oder Granulat) der Stoffzusammensetzung ab. In Ausführungsformen der Erfindung, die pro Gramm ca. $1x10^8$ KBE der probiotischen Mikroorganismen enthalten, sollten somit ca. $1x10^6$ KBE der probiotischen Mikroorganismen den Darm erreichen. Es hat sich gezeigt, dass mindestens ca. $1x10^6$ KBE probiotischen Mikroorganismen den Darm erreichen sollten, damit ein gesundheitsfördernder Effekt erzielt werden kann.

In Ausführungsformen der erfindungsgemäßen Stoffzusammensetzung enthält diese nach 18-monatiger Lagerung bei Raumtemperatur pro Gramm mindestens $1x10^8$ KBE der Probiotika. Hierdurch sollte sichergestellt werden, dass bei oraler Einnahme der Stoffzusammensetzung nach der Magenpassage mindestens ca. $1x10^6$ KBE der Probiotika den Darm erreichen.

Es hat sich gezeigt, dass die Lagerung der erfindungsgemäßen Stoffzusammensetzung einen Einfluss auf die Anzahl der enthaltenen lebenden Mikroorganismen hat. Bei einer Lagerung von ca. 12 Monaten bei Raumtemperatur kann es zu einer Abnahme der KBE Zahl der in der erfindungsgemäßen Stoffzusammensetzung enthaltenen probiotischen Mikroorganismen um durchschnittlich ca. zwei Zehnerpotenzen kommen. Die genaue Abnahme hängt, wie dem Fachmann bekannt ist, u.a. von der genauen Zusammensetzung der erfindungsgemäßen Stoffzusammensetzung sowie der Verabreichungsform bzw. Formulierung (z.B. Tablette, Kapsel, Pulver oder Granulat) der Stoffzusammensetzung ab. Entsprechend ist es vorteilhaft, dass die erfindungsgemäße Stoffzusammensetzung pro Gramm mindestens $1x10^{10}$, oder besser $1x10^{11}$ KBE der Probiotika enthält, damit nach Lagerung und Magenpassage noch mindestens $1x10^6$ KBE den Darm erreichen. Da die gesundheitsfördernde Wirkung der Stoffzusammensetzung stärker ist, wenn mehr als $1x10^6$ KBE der Probiotika den Darm erreichen, sind Stoffzusammensetzungen mit pro Gramm höheren KBE Zahlen im Bereich von $1x10^{12}$- $1x10^{17}$ besonders bevorzugt. Um keine negativen Effekte durch die Verabreichung einer zu großen Anzahl probiotischer Mikroorganismen zu erzeugen, sollte die Zahl der in der Stoffzusammensetzung pro Gramm enthaltenen KBE der Probiotika aber nicht deutlich über $1x10^{18}$ liegen.

In Ausführungsformen enthält die erfindungsgemäße Stoffzusammensetzung pro Gramm $1x10^6$ - $1x10^{19}$, $2x10^6$ - $5x10^{18}$, $5x10^6$- $2x10^{18}$, $1x10^7$-$1x10^{18}$, $2x10^7$- $5x10^{17}$, $5x10^7$- $2x10^{17}$, $1x10^8$-$1x10^{17}$, $2x10^8$- $5x10^{16}$, $5x10^8$- $2x10^{16}$, $1x10^9$- $1x10^{16}$, $2x10^9$- $5x10^{15}$, $5x10^9$-$2x10^{15}$, $1x10^{10}$-$1x10^{15}$, $2x10^{10}$ - $5x10^{14}$, $5x10^{10}$ - $2x10^{14}$, $1x10^{11}$ - $1x10^{14}$, $2x10^{11}$ - $5x10^{13}$, $5x10^{11}$ - $2x10^{13}$, $1x10^{12}$- $1x10^{13}$ oder $2x10^{12}$ - $5x10^{12}$ Kolonie-bildende Einheiten (KBE) der probiotische Mikroorganismen.

In Ausführungsformen der Erfindung enthält die Stoffzusammensetzung keinen Füllstoff. In anderen Ausführungsformen enthält die Stoffzusammensetzung mindestens einen Füllstoff. In Ausführungsformen ist der Anteil der Füllstoffe 0,01 - 20 Gew.-%, bevorzugt 2-17 Gew.-%, besonders bevorzugt 5-15 Gew.-%. In Ausführungsformen der Erfindung ist der Anteil der Füllstoffe 0,01 - 40 Gew.-%, 0,1 - 35 %, 0,1 - 40 %, 0,2 - 35 %, 0,3 - 30 %, 0,4 - 28 %, 0,5 - 26%, 0,6-24%, 0,8-22%, 1,0-20%, 1,2-19%, 1,4-18%, 1,6-17%, 1,8-16%,2,0-15 %, 2,5 -14 %, 3,0 -13 %,3,5-12 %, 4,0 -11%,4,5-10 %, 5,0 - 9,5 %, 5,5- 9,0 %, 6,0 - 8,5 %, 6,5 - 8,0 % oder 7,0 - 7,5 Gew.-%.

Ein Füllstoff ist ein Stoff, der die die Wirkung der erfindungsgemäßen Stoffzusammensetzung auf das Individuum, das die Stoffzusammensetzung einnimmt, nicht negativ beeinträchtigt und zudem keine negativen Auswirkungen auf die Wechselwirkung von den in den Stoffzusammensetzung enthaltenen Probiotika und Präbiotika hat. Zudem können Füllstoffe Gelbildende Wirkung haben, die beispielsweise stabilisierend auf die Stoffzusammensetzung in Tablettenform wirkt und die Herstellung von erfindungsgemäßen Tabletten mit höherer Härte und/oder Beständigkeit im Magenmilieu ermöglicht. Darüber hinaus können Füllstoffe als Trennmittel oder Schmiermittel genutzt werden, die die Tablettenpressung von pulverförmigen Stoffgemischen erleichtern und ein lautes Quietschen beim Pressen der Tablette verhindern.

In bevorzugten Ausführungsformen umfasst der mindestens eine Füllstoff Carnaubawachs, Palmfett, Maissiruppulver, Reisextrakt und/oder Silicium Dioxid. Carnaubawachs, Palmfett, Maissiruppulver, Reisextrakt und Silicium Dioxid sind vorteilhafte Füllstoffe, da diese in Bio-Quailität erhältlich sind bzw. der Bioqualität einer erfindungsgemäßen Zusammensetzung nicht entgegenstehen und zudem keine Milchbestandteile oder Industriezucker enthalten. Zudem sind Carnaubawachs, Palmfett, Maissiruppulver, Reisextrakt und Silicium Dioxid frei von tierischen Bestandteilen, was es ermöglicht, sie in einer veganen Stoffzusammensetzung zu verarbeiten. Darüber hinaus sind Carnaubawachs, Palmfett, Maissiruppulver, Reisextrakt und Silicium Dioxid Gluten-frei, was vorteilhaft für Allergiker ist, die die erfindungsgemäße Stoffzusammensetzung einnehmen.

Gemäß der Erfindung umfasst die Stoffzusammensetzung die Probiotika Lactobazillen und Bifidobakterien.

Lactobacillus (Plural: Laktobazillen) ist der Name einer Gattung von grampositiven, meist stäbchenförmigen Bakterien aus der Familie der Lactobacillaceae. Lactobacillus gehört zu den Milchsäurebakterien und erzeugt wie andere Milchsäurebakterien durch Gärung Milchsäure.

Bifidobakterien (identisch mit der Bakteriengattung Bifidobacterium) gehören zur Familie Bifidobacteriaceae und sind grampositive, sich nicht aktiv bewegende, nicht-Sporen bildende, überwiegend anaerobe Stäbchenbakterien. Sie bilden auf Agargel-Nährböden glatte Mikrokolonien ohne Filamente. Ihre Mitglieder besitzen Ähnlichkeit mit Corynebakterien. Zur normalen Flora von Magen-Darm-Trakt, Appendix und Vagina gehören u. a. Bifidobacterium bifidum, Bifidobacterium adolescentis, Bifidobacterium breve, Bifidobacterium longum und Bifidobacterium infantis. Sie sind nicht pathogen, d. h., sie sind für den Menschen unbedenklich.

Die Verwendung von Milchsäurebakterien der Gattungen Lactobacillus und Bifidobacterium in der erfindungsgemäßen Zusammensetzung ist besonders vorteilhaft, da die Wirksamkeit und Verträglichkeit dieser probiotischen Bakterien bereits nachhaltig belegt wurde.

Überraschenderweise hat sich gezeigt, dass die gesundheitsfördernde Wirkung dieser Bakteriengattungen noch gesteigert werden kann, wenn Sie zusammen mit mindestens zwei verschiedenen Präbiotika verabreicht werden. Es scheint, dass durch die Interaktion der Präbiotika und der Lactobazillen und/oder Bifidobakterien die Überlebensfähigkeit der Bakterien nach oraler Einnahme erhöht wird, so dass eine erhöhte Anzahl den Darm erreicht. Zudem können sich diese Bakterien aufgrund der Bereitstellung der Präbiotika direkt nach Erreichen des Darms sehr effizient replizieren und so einen besonders großen Effekt auf die Wirtsgesundheit und das Wohlbefinden ausüben. Es war sehr überraschend, dass dieser synergistische Effekt zwischen diesen probiotischen Milchsäurebakterien und den Präbiotika deutlich höher ausfällt, wenn mehr als ein Präbiotikum in der erfindungsgemäßen Stoffzusammensetzung enthalten ist.

So ist das Überleben der Bakterien im Magen spezifisch erhöht, wenn sie zusammen mit mehreren Präbiotika verabreicht werden im Vergleich zur Verabreichung mit nur einem einzelnen Präbiotikum.

Die Stoffzusammensetzung der Erfindung umfasst Lactobazillen und Bifidobakterien. Es hat sich gezeigt, dass durch die gemeinsame Verabreichung von probiotischen Bakterien beider Stämme synergistische Effekte in Bezug auf die Ausbereitung der Bakterien im Darm des Wirts erzielt werden können. Es ist überraschenderweise zu beobachten, dass die Besiedelung des gesamten Darms mit beiden Bakteriengattungen innerhalb weniger Tage nach Verabreichung dieser Ausführungsform der erfindungsgemäßen Stoffzusammensetzung festzustellen ist. Im Gegensatz dazu ist bei der Verabreichung einer Stoffzusammensetzung, die eine entsprechende Menge von entweder nur Lactobazillen oder Bifidobakterien enthält, zunächst nur zu einer lokalisierten Vermehrung der probiotischen Bakterien im Duodenum zu detektieren.

In bevorzugten Ausführungsformen der erfindungsgemäßen Stoffzusammensetzung produzieren die Probiotika rechtsdrehende Milchsäuren. Es hat sich gezeigt, dass die gesundheitsfördernde Wirkung der Stoffzusammensetzung besonders ausgeprägt ist, wenn die enthaltenen Mikroorganismen rechtsdrehende Milchsäure produzieren. Bevorzugt handelt es sich bei diesen Mikroorganismen um Lactobazillen oder Bifidobakterien. Diese Milchsäurebakterien bauen Zucker zu Milchsäure (Laktat) um. Milchsäurebakterien benötigen daher auch stets Kohlenhydrate zur Energiegewinnung. Im Rahmen der Erfindung werden diese Kohlenhydrate bevorzugt durch genau auf die jeweiligen Bakterien abgestimmte Kohlenhydrat-haltige Präbiotika bereitgestellt, so dass das Ausgangsprodukt für die Milchsäureproduktion durch die probiotischen Milchsäurebakterien gleich mitgeliefert wird. Allerdings kann der menschliche Körper nur rechtsdrehende Milchsäure (L-Lactat) umsetzen, weshalb der durch die Milchsäureproduktion erzielte positive Effekt vornehmlich dann gegeben ist, wenn die in der erfindungsgemäßen Stoffzusammensetzung enthaltenen Probiotika rechtsdrehende Milchsäuren produziert.

Offenbart ist eine Stoffzusammensetzung, die mindestens ein Probiotikum ausgewählt aus einer Gruppe umfassend Lactobacillus plantarum, Lactobacillus acidophilus, Lactobacillus paracasei, Bifidobacterium longum, Bifidobacterium lactis, Bifidobacterium bifidum und Bifidobacterium brevis enthält.

Offenbart (aber nicht Teil der Erfindung) ist eine Stoffzusammensetzung, die mindestens ein Probiotikum ausgewählt aus einer Gruppe umfassend Lactobacillus plantarum, Lactobacillus acidophilus, Lactobacillus paracasei, Bifidobacterium longum, Bifidobacterium lactis und Bifidobacterium bifidum enthält.

Offenbart (aber nicht Teil der Erfindung) wird eine Stoffzusammensetzung, die mindestens ein Probiotikum ausgewählt aus der Gruppe umfassend Lactobacillus plantarum, Lactobacillus acidophilus und Lactobacillus paracasei, und mindestens ein Probiotikum ausgewählt aus der Gruppe umfassend Bifidobacterium longum, Bifidobacterium lactis und Bifidobacterium bifidum enthält.

Erfindungsgemäße Stoffzusammensetzungen umfassen Lactobacillus plantarum, und dieser Bakterienstamm zeigt ein besonders gutes Wachstum in Gegenwart von Präbiotika. Insbesondere vermehrt sich Lactobacillus plantarum gut in Gegenwart von Baobab, Inulin, Agaveninulin, Pektin, Apfelfaser, Topinambur und/oder Konjak.

Erfindungsgemäße Stoffzusammensetzungen umfassen Lactobacillus paracasei, und dieser Bakterienstamm zeigt ein besonders gutes Wachstum in Gegenwart von Präbiotika. Insbesondere vermehrt sich Lactobacillus paracasei gut in Gegenwart von Baobab, Inulin, Agaveninulin, Pektin, Apfelfaser, Topinambur und/oder Konjak.

Erfindungsgemäße Stoffzusammensetzungen umfassen Bifidobacterium lactis, und dieser Bakterienstamm zeigt ein besonders gutes Wachstum in Gegenwart von Präbiotika. Insbesondere vermehrt sich Bifidobacterium lactis gut in Gegenwart von Baobab, Inulin, Agaveninulin, Pektin, Apfelfaser, Topinambur und/oder Konjak.

Die Stoffzusammensetzung der Erfindung umfasst die Probiotika Lactobacillus plantarum, Lactobacillus acidophilus, Lactobacillus paracasei, Bifidobacterium lactis und Bifidobacterium longum. Es hat sich gezeigt, dass diese Kombination von probiotischen Bakterien zu einer überraschend starken Vermehrung der enthaltenen Bakterien führt, die die Vermehrung der Bakterien aus Stoffzusammensetzungen, die nur einen Teil dieser Bakterienstämme enthalten, übersteigt.

Erfindungsgemäße Stoffzusammensetzungen umfassen Bifidobacterium bifidum und dieser Bakterienstamm zeigt ein besonders gutes Wachstum in Gegenwart von Präbiotika. Insbesondere vermehrt sich Bifidobacterium bifidum gut in Gegenwart von Baobab, Inulin, Agaveninulin, Pektin, Apfelfaser, Topinambur und/oder Konjak.

Die erfindungsgemäße Stoffzusammensetzung umfasst Lactobacillus plantarum, Lactobacillus paracasei und Bifidobacterium lactis. Es hat sich gezeigt, dass diese Kombination von probiotischen Bakterien zu einer überraschend starken Vermehrung der enthaltenen Bakterien führen, die die Vermehrung der Bakterien aus Stoffzusammensetzung, die nur einem oder zwei dieser Bakterienstämme enthalten, übersteigt.

Die Erfindung umfasst zudem Ausführungsformen, die mindestens ein Präbiotikum ausgewählt aus der Gruppe umfassend Topinambur, Baobab, Inulin, Glucomannan, Alginat, Agar-Agar, Guarkernmehl, Xanthan, Pektin, Dextrin, Cellulose, Lignin, Carrageen, Fruktooligosaccharide, Galactooligosaccharide und/oder Bestandteile hiervon enthalten. Bevorzugt enthält die erfindungsgemäße Stoffzusammensetzung mindestens ein Präbiotikum ausgewählt aus der Gruppe umfassend Topinambur, Baobab, Inulin, Konjakwurzel, Alginat, Agar-Agar, Guarkernmehl und/oder Xanthan.

Baobab ist die Frucht des Affenbrotbaums und die Verwendung von Baobab bzw. Baobab-Pulver in den Stoffzusammensetzungen der vorliegenden Erfindung hat sich als besonders positiv für herausgestellt, da Baobab sich gut in Flüssigkeiten löst und so insbesondere bei Verabreichung der Stoffzusammensetzung in Tablettenform das Wachstum der Bakterien nach Erreichen des Darms besonders gut fördern kann.

Die Verwendung von Inulin und Agaveninulin zur Herstellung der erfindungsgemäßen Stoffzusammensetzung ist mit mehreren überraschenden Vorteilen verbunden. So hat sich gezeigt, dass die Verpressung von pulverförmigen Stoffzusammensetzungen der Erfindung zu Tabletten in Gegenwart von Inulin zu besonders harten und gegenüber dem Magensaft resistenten Tabletten führen kann. Die Verwendung harter, Inulin-haltiger Tabletten macht es möglich, dass die Stoffzusammensetzung bei Erreichen des Darms noch einen überraschend hohen Anteil lebender Bakterien enthält und zudem die räumliche Nähe zwischen Probiotika und Präbiotika aufrecht erhalten werden kann. Diese räumliche Nähe ermöglicht eine Verstärkung des synergistischen Effekts zwischen diesen Komponenten der Stoffzusammensetzung. Aufgrund dieser überraschenden Funktionsweise des Inulins als Binder bei der Verpressung der erfindungsgemäßen Stoffzusammensetzung stellt Inulin eine besonders vorteilhafte Komponente dar. Inulin ist ein Gemisch von Polysacchariden aus Fructosebausteinen mit einer Kettenlänge von bis zu 100 Monomeren und einem endständigen Glucoserest. Es zählt zu den Fructanen und ist in verschiedenen Pflanzen enthalten, insbesondere in Arten der Korbblütler wie z.B. Yacon, Topinambur, Chicorée, Dahlie, Artischocke, Gewöhnlicher Löwenzahn, Schwarzwurzeln, aber auch Doldenblütler, z. B. die Pastinake, und in Agaven.

Apfelfaser ist ein Präbiotikum und Ballaststoff, der durch ein schonendes Produktionsverfahren aus entsafteten und getrockneten Äpfeln gewonnen wird und Pektin enthält. Die Verwendung von Apfelfaser in erfindungsgemäßen Stoffzusammensetzungen hat sich als vorteilhaft für die Wachstumsförderung der enthaltenen probiotischen Mikroorganismen erwiesen. Zudem hat sich gezeigt, dass die Beimischung von Apfelfasern die Mischfähigkeit der pulverförmigen Stoffzusammensetzung erhöht, so dass die verschiedenen Bestandteile gut verteilt sind innerhalbe der Mischung. Dies kann insbesondere wichtig für eine mögliche anschließende Tablettenpressung oder Verpackung in Kapseln sein, um sicherzustellen, dass eine homogene Mischung der Stoffzusammensetzung in die jeweilige Formulierungsvariante überführt wird.

Topinambur (Helianthus tuberosus) ist eine Pflanze und zählt botanisch zur Familie der Korbblütler (Asteraceae). Sie ist mit der Yacon (Smallanthus sanchifolius) verwandt und bildet wie diese essbare Wurzelknollen. Beide gehören zum Wurzelgemüse und sind Nutzpflanzen, deren Sprossknolle primär für die Ernährung genutzt wird. Die Verwendung von Topinambur bzw. Topinambur-Pulver ist vorteilhaft in erfindungsgemäßen Stoffzusammensetzungen, da dieses Präbiotikum sich besonders wachstumsfördernd auf fast alle Milchsäurebakterien, die Teil der Stoffzusammensetzung sein können, auswirkt und somit die Vermehrung der Bakterien im Darm besonders fördert. Dieser Effekt ist besonders ausgeprägt, und überraschend hoch, wenn die Stoffzusammensetzung in Tablettenform verabreicht wird und zudem Inulin enthält. Es hat sich überraschend gezeigt, dass Stoffzusammensetzungen, die Inulin und Topinambur enthalten die Herstellung von besonders harten Tabletten ermöglicht, wobei die Viabilität der enthaltenen Probiotika überraschenderweise kaum beeinträchtigt wird durch die Pressung bei hohem Druck. Topinambur Pulver enthält Inulin, das gegenüber Rein-Inulin ein kürzeres Kettenlängengemisch aufweist. Es hat eine sehr hohe mikrobiologische Aktivität in dem Sinn, dass es das Wachstum von präbiotischen Lactobazillen und Bifidobakterien besonders stark fördert. Der native Gehalt an physiologisch wertvollen Mineralstoffen wie Kalium, Kalzium Magnesium Phosphor ist besonders hoch in Topinamburpulver und die Mineralstoffaufnahme wird durch Topinambur verbessert.

Konjak bzw. die Konjakwurzel wird für die Herstellung von Konjakmehl genutzt. Die Konjakwurzel ist Teil der Teufelszunge (Amorphophallus Konjac), die besonders im Osten Asiens weit verbreitet ist. Konjak besteht zu 50 bis 60% aus Glucomannan, zu ca. 25% aus Stärke, zu 2 bis 5% aus Zellulose, zu 5 bis 10% aus Fett, zu 3 bis 5% aus Zucker und zu 3 bis 5% aus Mineralen. Glucomannan ist eine stärkeähnliche Substanz aus verzweigten Kohlenhydratketten. Die Hauptkette besteht dabei aus D-Mannose und D-Glucose, wobei in verschiedenen Abständen (kurze) Seitenketten auftreten.

Die Verwendung von Konjakwurzel und/oder Glucomannan als Präbiotikum im Rahmen der Stoffzusammensetzung der vorliegenden Erfindung hat den Vorteil, dass sich überraschenderweise gezeigt hat, dass diese zur Bildung eines Gels bei der Verpressung der Stoffzusammensetzung zu einer Tablette führt. Dies ermöglicht die Herstellung von Tabletten, die gegenüber dem Magensaft und Flüssigkeiten im Allgemeinen für einen überraschend langen Zeitraum resistent sind, auch wenn sie mit durchschnittlichem Pressdruck hergestellt werden. Diese überraschende Entdeckung wirkt sich sehr vorteilhaft für die vorliegende Erfindung aus, da erfindungsgemäße Tabletten gegenüber dem Magensaft sehr resistent sind und somit ihre Form und Zusammensetzung nach der oralen Einnahme bis zum Übergang in den Darm beibehalten, so dass erst dort die enthaltenen Probiotika freigesetzt werden. Die Magenpassage ist somit für das Überleben der in den entsprechenden Tabletten enthaltenen probiotischen Mikroorganismen weniger schädlich und die Probitika befinden sich beim Eintritt in den Darm weiterhin in der Nähe der Präbiotika, die das Wachstum und die Vermehrung der Probiotika dort besonders gut fördern können.

Der Begriff "Pektin" bezeichnet pflanzliche Polysaccharide, genauer Polyuronide, die im Wesentlichen aus $\alpha$-1,4-glycosidisch verknüpften D-Galacturonsäure-Einheiten bestehen. Ernährungsphysiologisch betrachtet sind Pektine Präbiotika und Ballaststoffe, da sie vom Menschen nicht direkt umgesetzt werden können, aber viele Mikroorganismen und insbesondere Probiotika in der Lage sind, Pektine in ihrem Stoffwechsel zu verwerten. Im Rahmen der vorliegenden Erfindung hat die Verwendung von Pektinen den Vorteil, dass diese sich stabilisierend auf Tablettenformulierungen der erfindungsgemäßen Stoffzusammensetzung auswirken und somit besonders Bruchfeste und/oder Magensaft-resistente Tabletten hergestellt werden können.

Chia Samen können aus der mexikanischen Chiapflanze (Salvia hispanica) gewonnen werden, die zu der Gattung des Salbei (Salvia) innerhalb der Familie der Lippenblütler (Lamiaceae) zählt. Chia Samen können für die Herstellung von Chia-Mehl genutzt werden und sind überdurchschnittlich reich an Omega-3-Fettsäuren, Antioxidantien, Proteinen, Ballaststoffen, Vitaminen und Mineralstoffen. Chia Samen und Chia-Mehl stellen Präbiotika im Sinne der Erfindung dar.

Agar (oder auch Agar-Agar, Agartang, Japanischer Fischleim, Kanten (japanisch), oder auch Chinesische oder Japanische Gelatine genannt) ist ein Galactose-Polymer (ein Polysaccharid), das Gallerte bilden kann. Die Grundeinheiten des Agars sind Agarose und sulfatiertes Agaropektin. Agar wird aus den Zellwänden einiger Algenarten (vor allem Rotalgen, wie Gracilaria-, Gelidiopsis-, Gelidium-, Hypnea- und Sphaerococcus-Arten), hauptsächlich aus Ostasien, hergestellt und stellt ein Präbiotikum im Sinne der vorliegenden Erfindung dar. Die Verwendung von Agar-Agar in der erfindungsgemäßen Stoffzusammensetzung ist vorteilhaft, da es bei der Formulierung der Stoffzusammensetzung als Tablette aufgrund seiner Gel-bildenden Eigenschaften dazu beitragen kann, dass die Stoffzusammensetzung zu sehr stabilen Tabletten verpresst werden kann, die sich nicht im Magensaft auflösen. Hierdurch wird gewährleistet, dass nur ein geringer Teil der in der Stoffzusammensetzung enthaltenen Mikroorganismen bei der Magenpassage stirbt und eine große Anzahl lebender Probiotika in den Darm gelangt und dabei in unmittelbarer Umgebung der wachstumsfördernden Präbiotika ist.

Die gelbildenden Präbiotika Alginat und Guarkernmehl bieten ähnliche Vorteile wie Agar-Agar bei der Verarbeitung der erfindungsgemäßen Stoffzusammensetzung.

Als Alginate werden die Salze der Alginsäure (auch Algin genannt) bezeichnet. Algin wird von Braunalgen gebildet und stellt in Algen das strukturgebende Element der Zellwände dar. Algin wird für die Verwendung in der Lebensmittel-, Pharma-, und Kosmetikindustrie aus den Braunalgen extrahiert und Alginat findet vor allem als Verdickungs- oder Geliermittel Verwendung. Alginat ist ein Polysaccharid, bestehend aus den beiden Uronsäuren $\alpha$-L-Guluronsäure (GulUA) und $\beta$-D-Mannuronsäure (ManUA), welche 1,4-glycosidisch in wechselndem Verhältnis zu linearen Ketten verbunden sind.

Guarkernmehl (auch Guargummi) ist ein Präbiotikum und Lebensmittelzusatzstoff, der zur Gruppe der Polysaccharide gehört und als Verdickungs- oder Geliermittel in vielen Lebensmitteln verwendet wird. Guarkernmehl wird aus den Samen der Guarbohne (Cyamopsis tetragonoloba) gewonnen. Es wird durch Entfernung von äußeren Schichten und anschließendes Zermahlen der übrigen Teile der Pflanze hergestellt. Wie Agar-Agar und Alginat wirkt sich Guarkernmehl stabilisierend auf die erfindungsgemäßen Stoffzusammensetzungen in Tablettenform aus.

Xanthan ist ein natürlich vorkommendes Polysaccharid, das mit Hilfe von Bakterien der Gattung Xanthomonas aus zuckerhaltigen Substraten gewonnen wird und als Lebensmittelzusatzstoff mit der E-Nummer E 415 als Verdickungs- und Geliermittel eingesetzt werden kann. Xanthan ist für Bio-/Öko-Lebensmittel zugelassen. Es kann vom menschlichen Organismus nicht umgesetzt werden und wird daher zu den Ballaststoffen gezählt. Im Darm wird es teilweise von dort lebenden Mikroorganismen abgebaut, weshalb es als Präbiotikum im Sinne der vorliegenden Erfindung angesehen wird. Aufgrund seiner Gel-bildenden Eigenschaften kann Xanthan zur Stabilisierung der vorliegenden Stoffzusammensetzung beitragen und ist ein bevorzugtes Präbiotikum der vorliegenden Erfindung.

Dextrin, auch Stärkegummi oder Maltodextrin genannt, ist ein Stärkeabbauprodukt, das eine Molekülgröße im Bereich zwischen Oligosacchariden und Stärke hat. Üblicherweise kommen es in Form von weißem bzw. hellgelbem Pulver vor und wird hauptsächlich aus Weizen-, Kartoffel- und Maisstärke durch trockene Erhitzung (>150 °C) oder unter Säureeinwirkung gewonnen. Dextrine entstehen auch durch den enzymatischen Abbau von Stärke durch Amylase.

Cellulose ist der Hauptbestandteil pflanzlicher Zellwände und ist das häufigste Polysaccharid (Vielfachzucker). Sie ist unverzweigt und besteht aus mehreren hunderten bis zehntausenden β-D-Glucose-Molekülen (β-1,4-glycosidische Bindung) bzw. Cellobiose-Einheiten. In der Nahrungsmittel- und Pharmaindustrie wird Cellulose bzw. werden Cellulosederivate verwendet, z. B. als Verdickungsmittel, Trägerstoff, Füllstoff, Trennmittel, Überzugsmittel und Schaummittel. Als Lebensmittelzusatzstoff trägt Cellulose die Bezeichnungen E 460 bis E 466.

Lignine (lat. lignum "Holz") bilden eine Gruppe von phenolischen Makromolekülen, die sich aus verschiedenen Monomerbausteinen zusammensetzen. Es handelt sich um feste Biopolymere, die in die pflanzliche Zellwand eingelagert werden und dadurch die Verholzung der Zelle bewirken (Lignifizierung). In Lebensmitteln zählt Lignin zu den Ballaststoffen und wird aufgrund seiner Umsetzbarkeit durch probiotische Mikroorganismen als Präbiotikum eingestuft.

Carrageen ist die Sammelbezeichnung einer Gruppe langkettiger Kohlenhydrate, die in Rotalgenzellen vorkommen. Es handelt sich um lineare, anionische Hydrokolloide, die sich anhand ihrer chemischen Struktur unterscheiden lassen und unterschiedliche Eigenschaften aufweisen. Diese verschiedenen Typen unterscheiden sich durch den Anteil an Galactose und 3,6-Anhydrogalactose sowie über die Anzahl an Sulfatgruppen. In der Lebensmittelindustrie wird Carrageen als Geliermittel und Verdickungsmittel eingesetzt.

Oligofructose (auch als Fructooligosaccharid oder FOS bezeichnet) ist ein Mehrfachzucker, der zwischen 30 und 50 % der Süßkraft von Saccharose aufweist und daher auch als Zuckeraustauschstoff verwendet wird. Da Verdauungsenzyme sie nicht abbauen können, gehört sie zu den Ballaststoffen und wird als Präbiotikum zur Stimulierung gesundheitsfördernder Dickdarmbakterien für eine gesunde Darmflora zugesetzt. Fructooligosaccharide haben den Vorteil, dass Lebensmittel, die keine anderen Zucker (Mono- oder Disaccharide) als Fructooligosaccharid enthalten, trotz des süßen Geschmacks als "zuckerfrei" bezeichnet werden können. Oligofructose ist aus drei bis zehn 1,2-β-glykosidisch verbundenen Fructose-Einheiten aufgebaut (ähnlich Inulin, dessen Kette aus rund 35 Einheiten besteht).

Galactooligosaccharide (GOS) sind Präbiotika, die aus Lactose bzw. aus Milch isoliert werden können. GOS bestehen aus einer bis sieben Galactose- und einer Glucose-Einheit.

Weitere Ausführungsformen der erfindungsgemäßen Stoffzusammensetzung umfassen mindestens ein Präbiotikum ausgewählt aus der Gruppe umfassend Topinambur, Baobab, Konjak, Glucomannan, Agaveninulin, Inulin, Agar-Agar, Alginat, Guarkernmehl und Xanthan.

Weitere Ausführungsformen der erfindungsgemäßen Stoffzusammensetzung umfassen mindestens zwei Präbiotika ausgewählt aus der Gruppe umfassend Topinambur, Baobab, Konjak, Glucomannan, Agaveninulin, Inulin, Agar-Agar, Alginat, Guarkernmehl und Xanthan.

Weitere Ausführungsformen der erfindungsgemäßen Stoffzusammensetzung umfassen mindestens drei Präbiotika ausgewählt aus der Gruppe umfassend Topinambur, Baobab, Konjak, Glucomannan, Agaveninulin, Inulin, Agar-Agar, Alginat, Guarkernmehl und Xanthan.

Weitere Ausführungsformen der erfindungsgemäßen Stoffzusammensetzung umfassen mindestens vier Präbiotika ausgewählt aus der Gruppe umfassend Topinambur, Baobab, Konjak, Glucomannan, Agaveninulin, Inulin, Agar-Agar, Alginat, Guarkernmehl und Xanthan.

Weitere Ausführungsformen der erfindungsgemäßen Stoffzusammensetzung umfassen mindestens fünf Präbiotika ausgewählt aus der Gruppe umfassend Topinambur, Baobab, Konjak, Glucomannan, Agaveninulin, Inulin, Agar-Agar, Alginat, Guarkernmehl und Xanthan. Eine weitere Ausführungsform der erfindungsgemäßen Stoffzusammensetzung umfasst die Präbiotika Topinambur, Baobab, Inulin und Agar-Agar.

Überraschenderweise hat sich gezeigt, dass Stoffzusammensetzungen, die mindestens diese vier Präbiotika enthalten, die Herstellung von besonders harten und haltbaren Tabletten ermöglichen. Dies ist insbesondere darum vorteilhaft, da sich entsprechende Tabletten nicht so schnell in Flüssigkeiten, und überraschenderweise auch nicht in sauren Flüssigkeiten, wie dem Magensaft, auflösen. Entsprechende Tabletten bleiben lange in ihrer Struktur erhalten und können so die enthaltenen probiotischen Mikroorganismen vor dem sauren Magenmilieu abschirmen und bewahren die räumliche Nähe zwischen den Probiotika und den schützenden bzw. stabilisierenden Präbiotika.

In einer bevorzugten Ausführungsform der vorliegenden Erfindung umfassen die Probiotika Lactobacillus plantarum, Lactobacillus acidophilus, Lactobacillus paracasei, Bifidobacterium lactis und Bifidobacterium longum, und die Präbiotika umfassen Topinambur, Baobab, Glucomannan und Inulin.

In einer weiteren bevorzugten Ausführungsform der vorliegenden Erfindung umfassen die Probiotika Lactobacillus plantarum, Lactobacillus acidophilus, Lactobacillus paracasei, Bifidobacterium lactis und Bifidobacterium longum, und die Präbiotika umfassen Topinambur, Baobab, Agar-Agar und Inulin.

In einer weiteren bevorzugten Ausführungsform der vorliegenden Erfindung umfassen die Probiotika Lactobacillus plantarum, Lactobacillus acidophilus, Lactobacillus paracasei, Bifidobacterium lactis und Bifidobacterium longum, und die Präbiotika umfassen Topinambur, Baobab, Agar-Agar, Glucomannan und Inulin.

Offenbart (aber nicht Teil der Erfindung) sind Stoffzusammensetzungen umfassend die Probiotika Lactobacillus plantarum, Lactobacillus paracasei, Bifidobacterium lactis und Bifidobacterium longum, und die Präbiotika Topinambur, Baobab, Agar-Agar und Inulin.

Offenbart (aber nicht Teil der Erfindung) sind Stoffzusammensetzungen umfassend die Probiotika Lactobacillus plantarum, Lactobacillus paracasei, Bifidobacterium lactis und Bifidobacterium longum, und die Präbiotika Topinambur, Baobab, Glucomannan und Inulin.

Offenbart (aber nicht Teil der Erfindung) sind Stoffzusammensetzungen umfassend die Probiotika Lactobacillus plantarum, Lactobacillus paracasei und Bifidobacterium bifidum, und die Präbiotika Topinambur, Baobab, Glucomannan,Agar-Agar und Inulin.

Bevorzugte Ausführungsformen der erfindungsgemäßen Stoffzusammensetzungen sind in Tabelle 1A dargestellt.

*Tabelle 1A: Bevorzugte Rezepte für die erfindungsgemäße Stoffzusammensetzung. Die Mengenverhältnisse sind in % angegeben. Dem Fachmann ist klar, dass die dargestellten Mengenverhältnisse variieren können.*

| **Stoffe** | **Rez 1** | **Rez 2** | **Rez 3** | **Rez 4** | **Rez 5** | **Rez 6** | **Rez 7** | **Rez 8** |
|---|---|---|---|---|---|---|---|---|
| **Probiotika** | 0,3 | 0,3 | 0,3 | 0,3 | 0,3 | 0,3 | 0,3 | 0,3 |
| **Topinambur** | 22,5 | 17,5 | 20 | 20 | 15 | 20 | 20 | 20 |
| **Baobab** | 22,5 | 17,5 | 30 | 29 | 29 | 29 | 20 | 20 |
| **Konjakwurzelpulver oder Glucomannan** | - | 1 | - | 1 | 1 | 1 | 0 | 1 |
| **Inulin oder Agaveninulin** | 29,7 | 33,7 | 32,2 | 32,2 | 27,2 | 32,2 | 29,7 | 28,7 |
| **Agar-Agar** | 15 | 25 | - | - | - | - | 15 | 15 |
| **Algina.** | - | - | 15 | 15 | - | - | | |
| **Cuarkernmehl** | - | - | - | - | 15 | 10 | | |
| **Xanttan (200 mesh)** | - | - | - | - | 10 | 5 | | |
| **Patmfett und/oder Camaubawachs** | - | - | 2,5 | 2,5 | 2,5 | 2,5 | - | |
| **Reisextrakt** | 3 | 2 | | | | | 5 | 5 |
| **Maissiruppulver** | 5 | 3 | | | | | 7 | 7 |
| **Silicium Dioxid** | 2 | 1 | | | | | 3 | 3 |
| **Summe (Gew.-%)** | 100 | 100 | 100 | 100 | 100 | 100 | 100 | 100 |

Es ist bevorzugt, dass die erfindungsgemäße Stoffzusammensetzung aus Bestandteilen in Bio-Qualität besteht. Bevorzugt besteht die erfindungsgemäße Stoffzusammensetzung aus natürlichen Produkten und umfasst keine industriellen oder künstlichen Inhaltsstoffe.

In Ausführungsformen ist die Stoffzusammensetzung dadurch gekennzeichnet, dass die Stoffzusammensetzung aus Bestandteilen in Bio-Qualität gemäß EG-Öko-Verordnung Nr. 834/2007 besteht, in ihrer Gesamtheit Bio-Qualität gemäß EG-Öko-Verordnung Nr. 834/2007 aufweist und/oder in ihrer Gesamtheit Bio-Qualität gemäß den Richtlinien des United States of America National Organic Program (USDA Organic) aufweist.

Erfindungsgemäße Stoffzusammensetzungen die in ihrer Gesamtheit Bio-Qualität gemäß EG-Öko-Verordnung Nr. 834/2007 aufweisen müssen nicht ausschließlich aus Bestandteilen in Bio-Qualität bestehen, sondern können gemäß der Richtlinien auch geringe Anteile andere Stoffe umfassen, um dennoch gemäß EG-Öko-Verordnung Nr. 834/2007 in ihrer Gesamtheit Bio-Qualität zu haben.

Die Begriffe "Bio-Qualität" oder "natürliche Bestandteile" im Sinne der Erfindung sind definiert gemäß der EG-Öko-Verordnung Nr. 834/2007 des Rates vom 28. Juni 2007 über die ökologische/biologische Produktion und die Kennzeichnung von ökologischen/biologischen Erzeugnissen (Europäische Öko-Verordnung oder EG-Öko-Verordnung), die definiert, wie Erzeugnisse oder Lebensmittel, die als Öko- bzw. Bio-Produkte gekennzeichnet sind, erzeugt und hergestellt werden müssen. Alternativ oder gleichzeitig kann können sich die Begriffe "Bio-Qualität" oder "natürliche Bestandteile" auf die Richtlinien des United States of America National Organic Program (USDA Organic) beziehen. Der Fachmann kann anhand der genannten EG-Öko-Verordnung Nr. 834/2007 die Kriterien für Bio-Qualität im Sinne der Erfindung erkennen. Insbesondere die folgenden Kriterien müssen erfüllt sein:

Produkte in Bio-Qualität dürfen nicht zur Konservierung ionisierender Strahlung ausgesetzt werden, nicht durch und mit gentechnisch veränderte/n Organismen erzeugt werden, nicht mit Einsatz von synthetischen Pflanzenschutzmitteln erzeugt werden, nicht mit Hilfe von leicht löslichen mineralischen Düngern erzeugt werden, nicht mehr als 5 % konventionell erzeugte Bestandteile enthalten (begrenzt auf eine Liste ausdrücklich erlaubter Rohstoffe), keine Süßstoffe und Stabilisatoren sowie synthetische Farbstoffe, Konservierungsmittel und Geschmacksverstärker enthalten, nur in einer Positiv-Liste aufgeführte pflanzliche Verdickungsmittel, Backtriebmittel oder Emulgatoren enthalten.

Die ausschließliche Verwendung von Bio-Qualität für die Herstellung der erfindungsgemäßen Stoffzusammensetzung ist vorteilhaft, da sich gezeigt hat, dass dies zu einer erhöhten Anwender-Compliance und somit zu regelmäßiger oraler Einnahme führt, was wiederum die gesundheitsfördernde Wirkung steigert.

In einer weiteren bevorzugten Ausführungsform enthält die erfindungsgemäße Stoffzusammensetzung keine tierischen Bestandteile enthält. Bevorzugt ist die erfindungsgemäße Stoffzusammensetzung vegan.

In Ausführungsformen liegt die erfindungsgemäße Stoffzusammensetzung in Form einer Tablette mit einer Bruchfestigkeit von mindestens 16 Newton vor.

Die Bruchfestigkeit ist ein wichtiger Parameter für Beurteilung von Tabletteneigenschaften. Bei einer niedrigen Bruchfestigkeit ist die Tablette sehr instabil und zerfällt ggf. schon direkt bei der Einnahme im Mund. Andererseits führt eine zu hohe Tablettenhärte dazu, dass eine Tablette den gesamten Magen-Darm-Trakt passieren kann, ohne zu zerfallen und Ihre Inhaltsstoffe freizusetzen. Vorliegend konnte festgestellt werden, dass erfindungsgemäße Stoffzusammensetzung mindestens eine Bruchfestigkeit von 16 Newton aufweisen sollten, um sicherzustellen, dass der Großteil der Enthaltenen Probiotika erst im Darm freigesetzt wird und die Tablette sich nicht bereits bei der Magenpassage auflöst.

In Ausführungsformen der Erfindung hat die erfindungsgemäße Tablette eine Härte von 10 - 120 Newton, bevorzugt 20 - 100 Newton, besonders bevorzugt 40 - 80 Newton. Es hat sich gezeigt, dass bei einer Härte von 40 - 80 Newton eine besonders gute Balance zwischen Stabilität der Tablette bei der Magenpassage, und kontinuierlicher Freisetzung der enthaltenen Probiotika und Präbiotika während der Darmpassage besteht. Tabletten die eine Bruchfestigkeit von weniger als 10 Newton haben zerfallen bereits im Magen, so dass die Inhaltsstoffe dem Magensaft ungeschützt ausgesetzt sind. Überraschenderweise hat sich gezeigt, dass erfindungsgemäße Tabletten, die Gel-bildende und/oder Komponenten mit Bindefunktion enthalten, schon bei Tablettenhärten von ca. 20 Newton eine sehr gute Stabilität im Magenmilieu aufweisen.

Weiterhin hat sich gezeigt, dass Tabletten mit einer Härte von ca. 20 Newton oder mehr eine gegenüber weicheren Tabletten verbesserte Haltbarkeit bei langen Lagerzeiten aufweisen. Dies bezieht sich sowohl auf die in etwa gleichbleibende Härte der Tabletten, die weder merklich aufweichen noch wesentlich nachhärten, als auch auf die Lebensdauer der enthaltenen probiotischen Mikroorganismen.

In Ausführungsformen der Erfindung weist die erfindungsgemäße Tablette eine Härte von 1 - 200 Newton, 2 - 190 Newton, 3 - 180 Newton, 4 - 160 Newton, 5 - 150 Newton, 6 - 140 Newton, 7 - 135 Newton, 8 - 130 Newton, 9 - 125 Newton, 10 - 120 Newton, 12 - 115 Newton, 14 - 110 Newton, 15 -105 Newton, 16 - 100 Newton, 18 - 95 Newton, 20 - 90 Newton, 25 - 85 Newton, 30 - 80 Newton, 35 - 75 Newton, 40 - 70 Newton, 45 - 65 Newton, 50 - 60 Newton oder ca. 53 - 57 Newton auf.

In einer besonders bevorzugten Ausführungsform betrifft die Erfindung eine Stoffzusammensetzung zur Herstellung einer gesundheitsfördernden Darmflora, dadurch gekennzeichnet, dass die Stoffzusammensetzung

- mindestens $1x10^8$ KBE Probiotika umfassend Lactobacillus plantarum, Lactobacillus acidophilus, Lactobacillus paracasei, Bifidobacterium longum und Bifidobacterium lactis,
- Präbiotika umfassend Topinambur, Baobab, Agar-Agar und Inulin mit einem Anteil von 80-99,7 Gew.-%, und
- mindestens einen Füllstoff ausgewählt aus der Gruppe umfassend Reisextraktpulver und Maissiruppulver als Füllstoff mit einem Anteil von 0,5-8 Gew.-% enthält,

wobei die Stoffzusammensetzung

- keine tierischen Bestandteile enthält,
- aus Bestandteilen in Bio-Qualität besteht und/oder in ihrer Gesamtheit Bio-Qualität aufweist,
- und als Tablette mit einem Gewicht von nicht mehr als 800 mg und mit einer Bruchfestigkeit von mindestens 16 Newton vorliegt.

Weiterhin betrifft die Offenbarung (nicht Teil der Erfindung) ein Verfahren zur Herstellung einer tablettenförmigen Stoffzusammensetzung zum Aufbau einer gesundheitsfördernden Darmflora, umfassend die Schritte

- Herstellung einer homogenen Mischung umfassend

i. zwei oder mehr Präbiotika in Pulverform,
ii. zwei oder mehr Probiotika in Pulverform,
iii. ein oder mehr Füllstoffe,

- Pressen der Mischung zu einer Tablette mit einer Bruchfestigkeit von mindestens 16 Newton.

Bevorzugte Ausführungsformen der erfindungsgemäßen Stoffzusammensetzung und die mit diesen assoziierten Vorteile sind in gleicher Weise auf das erfindungsgemäße Verfahren zur Herstellung einer tablettenförmigen Stoffzusammensetzung zum Aufbau einer gesundheitsfördernden Darmflora anzuwenden und entsprechende bevorzugte Ausführungsformen des Herstellungsverfahren sind hiermit ebenfalls offenbart. Weiterhin sind alle bevorzugten Ausführungsformen der erfindungsgemäßen Stoffzusammensetzung und die assoziierten Vorteile insbesondere für die erfindungsgemäße Stoffzusammensetzung in Tablettenform offenbart.

## SPEZIELLE BESCHREIBUNG DER ERFINDUNG

Im Folgenden soll die Erfindung anhand von Figuren und Ausführungsbeispielen und Vergleichsbeispielen erläutert werden, ohne jedoch hierauf beschränkt zu sein.

### Figuren

Figur 1: Dreifachbestimmung (1.1-1.3) einer Probe im Spotting Schema niedrig verdünnt (stock, $10^{-1}$, $10^{-2}$) und hoch verdünnt ($10^{-3}$, $10^{-4}$, $10^{-5}$).

Figur 2: *Bifidobacterium longum* und *Lactobacillus acidophilus* mittels Spotting Assay auf diversen Medien (niedrig verdünnt)

Figur 3: Anzucht der Mischkultur auf diversen Medien (niedrig verdünnt) und der positiven (MRS) sowie negativen Kontrolle mittels Spotting Assay (carbonfree MRS)

Figur 4: Anzucht der Mischkultur auf Erbse und Hafer mittels Spotting Assay (niedrig verdünnt)

Figur 5: Anzucht der stark verdünnten Mischkultur auf MRS-Agar und Topinambur mittels Spotting Assay

Figur 6: Anzucht der Mischkultur auf einer Kombination aus Baobab, Topinambur und Inulin

Figur 7: Anzucht der Einzelkultur von Bifidobacterium bifidum auf einer Kombination aus Baobab, Inulin und Topinambur (BIT) sowie auf den einzelnen Präbiotika

Figur 8: Vergleich unterschiedlicher Tablettenhärten (6kP und 4 kP) und deren Einfluss auf die Zerfallszeit im Magenmilieu

Figur 9: Prozentuale Masseabnahme der unterschiedlichen Chargen über die Zeit im Magenmilieu (ohne Chargen 1-3 mit Chiamehl)

Figur 10: Absterben der Mischkultur über die Zeit und in Abhängigkeit der Formulierung im synthetischen Magensaft auf der Schüttelplatte simuliert

## Ausführungsbeispiele und Vergleichsbeispiele

### 1. Materialien

#### Probiotika

Es erfolgte eine Vorauswahl an probiotischen Stämmen aus dem FloraFIT-Sortiment von Danisco. Hierbei wurden ausschließlich S1-Stämme ausgewählt, die bevorzugt rechtsdrehende Milchsäuren synthetisieren und als Zugabe in Lebensmitteln einen erwiesenen gesundheitlichen Nutzen hervorrufen. Diese Stämme wurden anschließend weiter untersucht und schließlich mit den verwendeten Präbiotika in eine Tablette verpresst.

*Tabelle* 1: *Vorauswahl der probiotischen Stämme*

| Name | Stamm |
|---|---|
| *Lactobacillus plantarum* | **La-14** |
| *Lactobacillus acidophilus* | **Lp-115** |
| *Lactobacillus paracasei* | **Lpc-37** |
| *Lactobacillus brevis* | **Lbr-35** |
| *Bifidobacterium longum* | **Bl-04** |
| *Bifidobacterium lactis* | **Bl-05** |
| *Bifidobacterium bifidum* | **BB-06** |

**Präbiotika**

Zur Vorauswahl der präbiotischen Komponenten wurden verschiedene Rohstoffe natürlicher Herkunft auf rein pflanzlicher Basis ausgewählt. Die Präbiotika stammen ausschließlich aus ökologischem Landbau. Die Vorauswahl ist Tabelle 2 zu entnehmen.

*Tabelle* 2: *Übersicht der ausgewählten Probiotika*

| Bezeichnung | Bio [ja/nein] |
|---|---|
| Topinambur | ja |
| Baobab | ja |
| Konjak | ja |
| Erbsenballaststoff | ja |
| Haferfaser | ja |
| Apfelmehl | ja |
| Apfelfaser | ja |
| Agaveninulin | ja |

**Hilfs- und Füllstoffe**

Das Bio-Komplexsynbiotikum sollte idealerweise frei von Füll- und Hilfsstoffen sein. Bei der Pressung stellte sich heraus, dass Tabletten, die unter der Zugabe von Palmfett oder Carnaubawachs als Trennmittel hergestellt wurden, vorteilhafte Eigenschaften aufweisen. Palmfett und Carnaubawachs sind Bio zugelassen. Vergleichbare Produkte des Standes der Technik enthalten Füllstoffe wie Maltodextrin, Laktose oder Mikrokristalline Cellulose. Die hier dargestellten Vergleichsbeispiele und Beispiele der erfindungsgemäßen Stoffzusammensetzung sind frei von diesen Stoffen und enthalten keinen Industriezucker sowie keine Milchbestandteile und sind außerdem vegan und glutenfrei.

**2. Anzucht der Einzelkulturen**

Um die Ausgangskeimzahl der probiotischen Bakterien zu bestimmen, werden alle Stämme in Einzelkultur kultiviert. Dieser Wert dient als Grundlage für Rezepturberechnungen, die koloniebildenden Einheiten als Wert für die Viabilität. Die hier bestimmten Werte werden in den weiteren Versuchen als Referenz herangezogen.

*Tabelle 3: Rezeptur des verwendeten De Man, Rogosa und Sharpe Agars (MRS-Agars)*

| Stoffe | c **[g/L]** |
|---|---|
| MRS-Bouillon | 52 |
| Agar-Agar | 15 |

(fortgesetzt)

| Stoffe | c [g/L] |
|---|---|
| Cystein (Bifido) | 0,5 |

## Durchführung

Es wurden 100 mg der einzelnen Stämme eingewogen (Aktivität Lactobacillen: $10^{16}$ KBE/g, siehe *Ergebnisse*) und in 10 ml PBS Puffer rehydriert. Bei der Erstellung der Verdünnungsreihen wurden zu jeweils 900 $\mu$L PBS 100 $\mu$L der zu Beginn hergestellten Lösung hinzugegeben. Die Probengefäße wurden nach Lösungszugabe mit Hilfe eines Vortex Mixers und vor Entnahme der nächsten Probe à 100 $\mu$L mittels Pipette durchmischt, um eine homogene Verteilung der Mikroorganismen (MO) und somit eine Reproduzierbarkeit zu gewährleisten. Wie in Tabelle 4 dargestellt, wurde die eigentlich erste Verdünnung der Gefäße mit stock-solution gekennzeichnet. Dies soll nur als Anmerkung bezüglich der Berechnung der Ausgangsviabilität dienen.

Zur Erklärung von Tabelle 4:

$$0{,}1 \text{ g (KBE } 10^{16}\text{/g) in 10 ml PBS rehydrieren} \rightarrow \frac{10^{15} KBE}{10\ mL}$$

Davon Entnahme von 100 $\mu$L $\rightarrow$ $10^{13}$ KBE (stock solution) im ersten Reaktionsgefäß.

*Tabelle 4: Darstellung der Verdünnungsreihe der Lactobacillen*

| Gefäßnr. | Stufe $10^x$ | Zellzahl | In 100 $\mu$L |
|---|---|---|---|
| 1 | stock solution | $10^{13}$ | **$10^{12}$** |
| 2 | -1 | $10^{12}$ | **$10^{11}$** |
| 3 | -2 | $10^{11}$ | **$10^{10}$** |
| 4 | -3 | $10^{10}$ | **$10^{9}$** |
| 5 | -4 | $10^{9}$ | **$10^{8}$** |
| 6 | -5 | $10^{8}$ | **$10^{7}$** |
| 7 | -6 | $10^{7}$ | **$10^{6}$** |
| 8 | -7 | $10^{6}$ | **$10^{5}$** |
| 9 | -8 | $10^{5}$ | **$10^{4}$** |
| 10 | -9 | $10^{4}$ | **$10^{3}$** |
| 11 | -10 | $10^{3}$ | **$10^{2}$** |
| 12 | -11 | $10^{2}$ | **$10^{1}$** |
| 13 | -12 | $10^{1}$ | **$10^{0}$** |

Jeweils 100 $\mu$l der gewünschten Verdünnungsstufe wurden in eine Petrischale gegeben und mit "handwarmem" Agar vergossen. Wenn durch die Hitze kein Schmerzempfinden zustande kommt, ist die Temperatur genau richtig, um ein Absterben der Stämme auszuschließen. Es ist allerdings Vorsicht geboten, da das Temperaturfenster zwischen möglicher Anwendung und Festwerden des Agars nicht allzu groß ist. Daher sollten nicht mehr als zwanzig Platten in einem Zug vergossen werden. Damit sich die MO-Lösung gleichmäßig verteilt, wurde die Petrischale direkt nach Zugabe des noch flüssigen Agars in Achtern auf der Tischplatte geschwenkt. Anschließend wurden die Platten bei 37 °C für 48 h im Brutschrank inkubiert. Nach dem Auszählen der Kolonien konnte über die Verdünnungsstufe auf die Anfangsviabilität der Stämme geschlossen werden.

## Ergebnisse

Alle sieben verwendeten Stämme zeigen deutlich höhere Werte als vom Hersteller angegeben. Die genauen Werte sind den Tabellen 5 und 6 zu entnehmen.

*Tabelle 5: Keimzahlbestimmung der Lactobacillen in Einzelkultur nach 48 h bei 37 °C (Verdünnung $10^{-10}$ bis $10^{-12}$)*

| | -10 | | | | -11 | | | | -12 | | | |
|---|---|---|---|---|---|---|---|---|---|---|---|---|
| ***L. plantarum*** | 412 | 463 | 352 | 337 | 47 | 58 | 50 | 31 | 5 | 6 | 5 | 7 |
| Mittelwert | 391,00 | | | | 46,50 | | | | 5,75 | | | |
| ***L. acidophilus*** | | | | | 78 | 59 | 55 | 55 | 5 | 6 | 3 | 8 |
| Mittelwert | | | | | 61,75 | | | | 5,50 | | | |
| ***L. paracasei*** | 106 | 133 | 148 | 129 | 20 | 11 | 69 | 19 | | | | |
| Mittelwert | 129,00 | | | | 29,75 | | | | | | | |

Bei der Viabilitätsbestimmung der Bifidobacterien wurde zu der Standardmischung des MRS-Agars (MRS-Bouillon: 52 g/L; Agar-Agar: 15 g/L) Cystein zugegeben (0,5 g/L). Zusätzlich wurden die Bifidobacterien als strikt anaerobe MO in Anaerobiertöpfen unter Zugabe von Anaeropacks inkubiert. Die Lactobacillen benötigen als anaerotolerante Bakterien nicht zwingend ein anaerobes Milieu, weshalb diese auch als lose Platten im Brutschrank inkubiert werden können.

*Tabelle 6: Keimzahlbestimmung der Bifidobacterien in Einzelkultur nach 48 h bei 37 °C (Verdünnung $10^{-9}$ bis $10^{-12}$)*

| | -9 | | | | -10 | | | | -11 | | | | -12 | | | |
|---|---|---|---|---|---|---|---|---|---|---|---|---|---|---|---|---|
| ***B. longum*** | 210 | 176 | 164 | 143 | 19 | 22 | 18 | 20 | 5 | 3 | 6 | 8 | | | | |
| Mittelwert | 173,25 | | | | 19,75 | | | | 5,50 | | | | | | | |
| ***B. lactis*** | 368 | 465 | 385 | 221 | 33 | 39 | 24 | 14 | 3 | 5 | 5 | 4 | | | | |
| Mittelwert | 359,75 | | | | 27,50 | | | | 4,25 | | | | | | | |
| ***L. brevis*** | | | | | 600 | 750 | 354 | 800 | 168 | 78 | 96 | 41 | 8 | 22 | 4 | 11 |
| Mittelwert | | | | | 626 | | | | 95,75 | | | | 11,25 | | | |
| ***B. bifidum*** | 458 | 650 | 900 | 185 | 84 | 66 | 41 | 78 | 5 | 16 | 7 | 9 | | | | |
| Mittelwert | 548,25 | | | | 67,25 | | | | 9,25 | | | | | | | |

Die Ermittlung der in Tabelle 7 angegeben Viabilitäten erfolgte ein halbes Jahr nach dem Kauf der MO, also nach Ablauf der Hälfte des MHD. Hierbei wurden die Lactobacillen und Bifidobacterien in den Origanalverpackungen der Hersteller bei -80 °C gelagert.

*Tabelle 7: Vergleich der Herstellerangaben (MHD) mit den ermittelten Keimzahlen (Mibi)*

| | Anzahl/g (MHD) | Anzahl/g (Mibi) |
|---|---|---|
| ***L. plantarum*** | $4*10^{11}$ | $4,28*10^{16}$ |
| ***L. acidophilus*** | $2*10^{11}$ | $6,18*10^{16}$ |
| ***L. paracasei*** | $4*10^{11}$ | $2,13*10^{16}$ |
| ***B. longum*** | $1*10^{11}$ | $1,85*10^{15}$ |
| ***B. lactis*** | $5*10^{11}$ | $3,17*10^{15}$ |
| ***L. brevis*** | $2*10^{11}$ | $9,01*10^{16}$ |
| ***B. bifidum*** | $2*10^{11}$ | $7,15*10^{15}$ |

## 3. Anzucht der Mischkulturen

Bei Anzucht der Mischkultur wurde analog wie bei den Einzelkulturen vorgegangen, nur dass anstatt 0,1 g der jeweiligen Stämme auf 10 ml PBS lediglich 0,02 g des einzelnen Probiotikums zugegeben wurden. In der Summe ergaben alle fünf verwendeten Stämme dann jedoch wieder 0,1 g. Hierbei wurden die folgenden fünf Stämme hergenommen: *Lactobacillus plantarum, Lactobacillus paracasei, Lactobacillus acidophilus, Bifidobacterium lactis und Bifidobacterium longum.*

*Tabelle 10: Keimzahlbestimmung der Mischkultur nach 48 h bei 37 °C*

| | -10 | | | | -11 | | | | -12 | | | |
|---|---|---|---|---|---|---|---|---|---|---|---|---|
| *Mischkultur* | 2000 | 2000 | 1000 | 1500 | 423 | 577 | 941 | 136 | 71 | 85 | 42 | 61 |
| Mittelwert | 1625 | | | | 519,25 | | | | 64,75 | | | |

Auffällig war hier, dass die Mischkultur eine höhere Keimzahl aufweist als die der Einzelkulturen.

## 4. Löslichkeitsverhalten der Präbiotika

Es wurden jeweils 10 g des Präbiotikums abgewogen und in einen 250 ml Erlenmeyerkolben überführt. Anschließend wurden 90 g destilliertes Wasser hinzugegeben. Danach wurden die Gefäße mittels eines Rührfischs und Magnetrührers jeweils für 5 min vermengt. Die Löslichkeit wurde ausschließlich visuell evaluiert.

Bei der Untersuchung des Löslichkeitsverhaltens fiel auf, dass sich zwar zu Beginn die meisten Stoffe gut im Medium verteilen ließen, jedoch meistens nicht auflösten. Dies konnte an dem sich nach wenigen Minuten bildenden Sediment und klaren Überstand deutlich identifiziert werden. Um eine mögliche Sättigung durch eine zu große Zugabe auszuschließen, wurden ebenso Versuche mit jeweils 0,5 g Präbiotikum auf 500 ml Medium durchgeführt. Diese Versuchsdurchführung führte zu selben Ergebnissen. Das Agaveninulin war auch das einzige Präbiotikum, das als löslich bewertet wurde, wobei sich bei Baobab wenigstens kleine Anteile gelöst hatten.

*Tabelle 11: Löslichkeit der untersuchten Präbiotika*

| **Präbiotikum** | **Löslichkeit** | **optische Bewertung** |
|---|---|---|
| Baobab | z.T. löslich | + |
| Topinambur | unlöslich | - |

(fortgesetzt)

| Präbiotikum | Löslichkeit | optische Bewertung |
|---|---|---|
| Konjak | Gelbildner | bildet Gel |
| Erbse | unlöslich | - |
| Hafer | unlöslich | - |
| Apfelfaser | unlöslich | - |
| Apfelmehl | unlöslich | - |
| Agaveninulin | löslich | +++ |

**5. Fließfähigkeit**

Die Fließfähigkeit bestimmt nicht nur die Mischfähigkeit der Präbiotika untereinander, sondern ist auch eine bedeutende Größe, um den gleichmäßigen Fluss der Rezeptur durch den Zulauftrichter der Tablettenpresse zu gewährleisten, falls diese nicht mit einem Rührflügel ausgestattet ist. Als Messvorrichtung diente hierbei ein Edelstahltrichter, durch den das Pulver in einen Messzylinder rieseln konnte.

Es wurden jeweils 30 g der Präbiotika in den Trichter gegeben, dessen Öffnung verschlossen war. Nach Lösen des Stopfens rieselte das Pulver in den Zylinder, wodurch über Masse und abzulesendes Volumen die Schüttdichte bestimmt werden konnte.

$$\rho_B = \frac{m}{V_B}$$

$\rho_B$: Schüttdichte m: Masse $V_B$: Schüttvolumen

Daraufhin wurde mit Hilfe eines Stampfvolumeters die Stampfdichte bestimmt. Ein Stampfvolumeter ist ein Messgerät mit zwei Drehtellern zur Aufnahme von je einem Messzylinder ausgestattet. Nach der durchlaufenen Hubanzahl von 250 kann das Stampfvolumen am Zylinder abgelesen und somit die Stampfdichte bestimmt werden. Unter einem Hub versteht man hierbei das automatische Anheben des Drehtellers mit gefülltem Messzylinder mit anschließendem Herunterfallen, wodurch es zu einem Zusammenfallen des Pulverbetts kommt und das Probenvolumen durch die Kompaktierung entsprechend abnimmt.

$$\rho_T = \frac{m}{V_T}$$

$\rho_T$: Stampfdichte m: Masse $V_T$: Stampfvolumen

Abschließend konnte dadurch als Maß für die Fließfähigkeit der Pulvermischung der Hausner-Faktor bestimmt werden. Im Allgemeinen sollten die Werte der Schütt- und Stampfdichte nah aneinander liegen, da sich große Abweichungen unter anderem negativ auf die Dosiergenauigkeit auswirken. Trotz des einfachen Aufbaus findet dieses Verfahren der Bestimmung der Fließfähigkeit in der pharmazeutischen Industrie nach wie vor Anwendung.

Dabei spricht ein Hausner-Faktor größer als 1,5 für einen schlechten Fluss des Pulvers, während unter 1,25 liegende Werte eine gute Fließfähigkeit vertreten. Dazwischen befindliche Werte stellen einen moderaten Fluss dar.

$$H = \frac{\rho_T}{\rho_B}$$

H: Hausner-Faktor

*Tabelle 12: Untersuchung der Fließfähigkeit mittels des Hausner-Faktors*

| Stoff | m [g] | $V_B$ [ml] | $V_T$ [ml] | $\rho_B$ [g/ml] | $\rho_T$ [g/ml] | H |
|---|---|---|---|---|---|---|
| Baobab | 30 | 79 | 53 | 0,38 | 0,57 | 1,49 |
| Topinambur | 30 | 62 | 50 | 0,48 | 0,60 | 1,24 |
| Inulin | 30 | 54 | 41 | 0,56 | 0,73 | 1,30 |
| Apfelfaser | 30 | 60 | 49 | 0,50 | 0,61 | 1,22 |
| Hafer | 30 | 81 | 53 | 0,37 | 0,57 | 1,53 |
| Erbse | 30 | 90 | 55 | 0,33 | 0,55 | 1,64 |
| Konjak | 30 | 45 | 40 | 0,67 | 0,75 | 1,13 |
| Apfelmehl | 30 | 110 | 88 | 0,27 | 0,34 | 1,25 |

Aus Tabelle 12 ist zu schließen, dass Apfelfaser, Topinambur, Apfelmehl und Konjak die besten Fließeigenschaften aufweisen. Somit könnten nach Untersuchung der Fließfähigkeit Topinambur und Inulin das Grundgerüst der Rezeptur bilden. Dies steht auch in Einklang mit dem im Folgenden durchgeführten Spotting Assay zur Evaluierung der wachstumsfördernden Eigenschaften der ausgewählten Präbiotika.

**Mischen**

Da die zugesetzten Probiotika eine so geringe Meng im Vergleich zu den Präbiotika stellen, ist es wichtig, dass diese homogen in der Stoffzusammensetzung verteilt sind. Um dies zu gewährleisten ist es bevorzugt, dass die Partikelgrößen nicht zu stark variieren, die jeweiligen Präbiotika gut fließen und Vormischungen angesetzt werden.

Da erster und zweiter Punkt bei den verwendeten Präbiotika zutreffen, lag der Fokus beim Mischvorgang auf kleineren Vormischungen. Bei der jeweiligen Charge wurden die Probiotika mit dem am besten fließfähigen Konjak (Glucomannan) vermengt. Anschließend wurde Topinambur hinzugegeben. Dies ging weiter, bis die gesamte Mischung fertig war, vom am besten bis zum schlechtesten fließfähigen Pulver.

**6. Wechselwirkung Probiotikum - Präbiotikum**

Die zu verpressenden Komponenten sollen optimal aufeinander abgestimmt sein. Daher müssen Präbiotika Anwendung finden, die von den jeweiligen Mikroorganismen (MO) sehr gut metabolisiert werden können, also im Wachstumsmedium als Kohlenstoff-Quelle angenommen werden.

Eine optimale Abstimmung der Komponenten untereinander garantiert eine bessere Überlebensrate im Magen und damit eine gesteigerte Besiedelung des Darms, wodurch unerwünschte Keime verdrängt werden.

Zur Validierung der Ergebnisse wurden zwei Referenzen eingesetzt. Eine positive Referenz mit 2 % Glucose und eine negative Referenz auf cfMRS (carbonfree MRS). Das Wachstum der jeweiligen Stämme/Mischung wurde mittels Spotting Assay beobachtet.

**Spotting Assay**

*Plattenvorbereitung*

Es wurden drei verschiedene Arten von Medien vorbereitet:

- o Carbonfree MRS (siehe Tabelle 13)
- o Positivkontrolle 2 % Glucose (Fertignährmedium)
- o Medium mit Präbiotikum zu 2 %

*Tabelle 13: Zusammensetzung des C-freien MRS-Agar und dem entsprechenden Präbiotikum-Agar (fett und kursiv gedruckt)*

| **Substanz** | **c [g/L]** |
|---|---|
| Proteose Pepton No. 3 | 10,000 |
| Hefeextrakt | 5,000 |
| Diammoniumhydrogen-Citrat | 2,000 |
| Tween 80 | 1,000 |
| Magnesiumsulfat | 0,206 |
| Mangansulfat | 0,056 |
| Dikaliumhydrogenphosphat | 2,000 |
| Agar-Agar | 15,000 |
| ***Präbiotikum*** | ***20,000*** |

Zu jedem Medium wurden 15 g/L Agar-Agar hinzugegeben. Hergestellt wurden die Nährböden in 250 ml Schott-Flaschen, die jeweils nur bis zu 150 ml befüllt wurden, damit die Keimfreiheit beim Aufkochen in der Mikrowelle sichergestellt ist und das Medium gleichzeitig nicht übergeht.

Die Präbiotika können direkt zum Pulver des Mediums hinzugegeben und zusammen aufgekocht werden. Allerdings müssen die Platten dann anaerob kultiviert werden, da unter aeroben Bedingungen Probleme mit Sporenbildnern entstehen. Sollten die Platten dennoch fremdverkeimt sein, müssen die Präbiotika vorab abgekocht werden.

◦ 10 % Lösung in demin. $H_2O$ (10 g des jeweiligen Präbiotikums + 90 g Wasser)
o Mehrere Male in 500 ml Becherglas hoch aufkochen lassen, dann erst Zugabe zu Medium!
o Zugabe dieser Lösung mit 20 % zu Medium

→ insgesamt 2 % Präbiotikum im Nährboden

Das für die Bifidobakterien wichtige Cystein wurde normalerweise immer zu der MRS-Agar Pulvermischung direkt hinzugegeben. Hier ist es jedoch von Vorteil, auf den Nährböden 100 µl einer Cystein-Lösung zu verspateln, um ein gleichmäßiges Wachstum zu gewährleisten. Die Inkubation erfolgte bei 37 °C für 48 h. Pro Platte wurden 10 Spots wie in folgendem Schema verteilt (Figuren 1). Die Verdünnungen können Tabelle 4 entnommen werden. Neben einer PBS-Referenz, die bestenfalls auf den Platten nicht zu sehen sein sollte, wurden nach Figur 1 in drei Spalten drei verschiedene Verdünnungen verteilt (Dreifachbestimmung pro Verdünnung und Nährboden). Das Wachstum wurde mit allen Keimen (Einzelkultur und Mischkultur) auf allen Nährmedien, auf MRS-Agar sowie auf den vorgesehenen Präbiotika untersucht.

*Einzelkultur*

Hierbei zeigte sich, dass *bifidobacterium longum* auf allen Präbiotika-Medien als auch auf deren Kombination ein mäßiges Wachstum aufweist. Ebenso zeigt *lactobacillus acidophilus* nur ein mäßiges Wachstum. Es bildeten sich bei Ersterem kaum durchgehende Spots aus und oft waren eine Verdünnung nach der stock solution nur noch wenige kleine Punkte zu erkennen (Figur 2). Im Gegensatz dazu zeigten sich bei *lactobacillus acidophilus* zwar durchgängige Spots, allerdings waren diese sehr schwach im Vergleich zu den anderen Lactobacillus Stämmen.

Bei Lactobacillus brevis zeigte sich kein besonderes Wachstum. Die übrigen Stämme zeigten alle gutes Wachstum. Insbesondere Bifidobacterium bifidum zeigte ein sehr gutes Wachstum, sowohl besser als das von Bifidobacterium longum als auch von Bifidobacterium lactis (Figur 7).

*Mischkultur*

Bei der Anzucht der Mischkultur sollten bestmöglich stark ausgeprägte Spots zu sehen sein, die für ein gutes Wachstum der Stammkombination auf den Medien sprechen. Es wurden neben den einzelnen Präbiotika auch Proben auf MRS-Agar untersucht, um eine Referenz zu erhalten. Ein referenznahes Wachstum wäre ein Kriterium, das für die jeweiligen Präbiotika spricht.

Über die Versuche hinweg verdeutlichte sich, dass die Präbiotika mit festen großen Bestandteilen das Wachstum am besten fördern. Somit waren die Spots auf Topinambur und Apfelfaser durchgehend am kräftigsten. Wie Figur 3 zeigt, war auch generell zu sehen, dass die Spots auf Baobab sowie Inulin nicht nur etwas an Deckkraft verlieren, sondern auch teilweise in punktuelle Kolonien übergehen (Inulin, Verdünnung $10^{-2}$).

Das Wachstum auf Konjak hingegen wirkt auf den ersten Blick ebenso schwach, war aber durchgängig und beständig. Die Ergebnisse wurden hierbei vermutlich aufgrund der Schwierigkeit, dass Konjak recht schnell in Flüssigkeiten ein Gel bildet, beeinflusst. Dies erschwerte den gesamten Prozess des Plattengießens sowie des Aufbringens auf einer ungeraden Oberfläche. Doch es zeigte sich auch bei höheren Verdünnungen auf Konjak immer noch ein gutes Wachstum, welches mit dem des MRS-Agars mithalten konnte.

Dennoch waren die Ergebnisse im Großen und Ganzen besser als die auf den anderen evaluierten Medien Erbse und Hafer. Auf Erbse beispielsweise waren schon die stock solutions der Bifidobacterien kaum zu erkennen. In ähnlicher Weise wuchsen auch auf Hafer die Keime nur sehr zaghaft. Figur 4 zeigt das schwache Wachstum der Mischkultur auf Erbse und Hafer. Da diese beiden Präbiotika auch schon eine schlechte Fließfähigkeit aufgewiesen haben, wurden sie bei weiteren Versuchen nicht mehr berücksichtigt.

Im Nachfolgenden wurde noch ein weiterer Vergleich zwischen den wachstumsstärksten Präbiotika und einem MRS-Agar gezogen. Figur 5 demonstriert, dass sich bei Verdünnungen ab $10^{-5}$ selbst bei dem MRS-Agar kaum mehr Kolonien ausbilden können, während auf Topinambur ein vergleichsweise gutes Wachstum zu sehen ist. Gleiches Verhalten wurde auf Konjak und Apfelfaser beobachtet. Weitere Untersuchungen, die für die Verwendung der ausgesuchten Präbiotika sprechen, folgen in der Flüssigkultivierung. Figur 6 zeigt das Wachstum der Mischkultur auf einer Mischung der drei Hauptpräbiotika Topinambur, Inulin und Baobab. Dabei ist sehr gutes Wachstum auch bei einer Kombination zu erkennen.

**Flüssigkultivierung**

Um die Wechselwirkung der Pro- und Präbiotika weiter zu untersuchen, wurde die zu verwendende Mischkultur flüssig kultiviert. Dabei ging es vor allem darum, einen Vergleich zu dem als positive Kontrolle angesetzten MRS-Bouillon anzustellen.

*Durchführung*

Der Ansatz erfolgte in einem kleinen Becherglas, das mit Parafilm so verschlossen wurde, dass ein Fremdeintrag verhindert wurde, der Luftaustausch zur anaeroben Kultivierung aber noch stattfinden konnte. In jedes Becherglas wurden 10 ml PBS gefüllt. Dazu wurden 0,5 g des jeweiligen Mediums gegeben. Verwendet wurden hierbei MRS-Bouillon, PBS (Leerprobe), Baobab, Inulin, Apfelfaser, Topinambur, Konjak sowie Apfelpektin. Zusätzlich wurde eine Kultivierung in der Kombination der Hauptkomponenten Topinambur und Apfelfaser vorgenommen (jeweils 0,25 g). Anschließend wurden 100 µl der rehydrierten Mischkultur (0,02 g je Einzelkultur mit 10 ml PBS) aus dem Sterilgefäß zu den Bechergläsern hinzugegeben. Kultiviert wurde in einem 5 l Anaerobiertopf, in den zwei Anaeropacks hinzugegeben wurden. Beim Ausplattieren der Verdünnungsstufe $10^{-12}$ wäre somit eine Zellzahl von $10^{0}$ Zellen zu erwarten gewesen.

*Ergebnisse*

Im Fokus des Versuchs standen die Hauptkomponenten der Rezeptur Topinambur und Apfelfaser.

In Aussicht auf eine wünschenswerte, wachstumsfördernde Kultivierung wurden im ersten Versuch die Verdünnungsstufen $10^{-12}$ bis $10^{-14}$ ausplattiert. Hierbei zeigte sich auf den Platten, bei deren Flüssigkultivierung Baobab und Inulin verwendet wurden, ein relativ geringes Wachstum. Da Baobab und Inulin auch beim Spotting Assay lediglich ein passables Medium darstellten, entsprach dieses Ergebnis in etwa den Erwartungen. Es könnte sein, dass dies an der guten Löslichkeit des Inulins bzw. mäßiger Löslichkeit des Baobabs liegt. Aus folgenden Versuchen geht überraschenderweise hervor, dass die Mikroorganismen scheinbar feste Bestandteile besser verwerten.

Im folgenden Versuch wurden als Anzuchtmedium neben MRS-Bouillon, Apfelfaser und Topinambur auch eine Kombination aus Apfelfaser und Topinambur verwendet. Die Zugabe der Präbiotika führte im Vergleich zu MRS Bouillon zu einem verbesserten Wachstum (Tabelle 14). Dies demonstriert ein weiteres Mal, dass Topinambur und Apfelfaser einen optimalen Grundstock der Rezeptur bilden und gute Voraussetzungen für die Tablettenformulierung stellen.

Den Ergebnissen zufolge werden unlösliche Medien scheinbar grundsätzlich von den Probiotika effizienter verwertet.

*Tabelle 14: Darstellung der KBE nach der Flüssigkultivierung mit diversen Medien*

| | **Verdünnungsstufe** | | | | | | | | | |
|---|---|---|---|---|---|---|---|---|---|---|
| **Medium** | -17 | | -18 | | -19 | | -20 | | -21 | |
| M RS-Bouillon | 1200 | 1000 | 87 | 93 | 3 | 6 | | | | |
| Topinambur | | | | | 190 | 230 | 42 | 36 | 1 | 3 |
| Apfelfaser | | | | | 224 | 220 | 12 | 73 | 5 | 9 |
| TOP + AF | | | | | 215 | 210 | 91 | 18 | 5 | 0 |

## 7. Untersuchung der Tablettenformulierungen

Im Folgenden werden drei Pressversuche beschrieben und deren Ergebnisse ausgewertet. Häufig vorkommende Begrifflichkeiten werden hier kurz definiert.

*Gelbildner* sind Substanzen, die der Rezeptur beigemengt werden, dort als Gerüstbildner fungieren, indem sie eine flüssige Phase verdicken oder ein gummiartiges Gel ausbilden.

Als *Bindemittel* werden Stoffe bezeichnet, deren Aufgabe der Zusammenhalt der Tablette ist und die somit die Freisetzung der Inhaltsstoffe der Tablette beeinflussen.

Ein *Sprengmittel* ist ein Stoff, der wenig plastisch verformbar ist, am besten einen elastischen Anteil besitzt, der sich nach dem Pressen in seine alte Form zurückdrängt und dadurch die Tablette leicht zerfallen lässt.

Weiterhin ist ein *Trennmittel/Schmiermittel* dazu da, um die Rezeptur zu "schmieren", so dass die Pulvermischung, ohne ein lautes Quietschen zu erzeugen, verpresst werden kann.

Ebenso wird von Rezepturen sowie Chargen gesprochen. Eine *Rezeptur* ist die Zusammensetzung des Pulvergemisches aus verschiedenen Anteilen, wobei eine *Charge* die schon verpresste Zusammensetzung darstellt, also die fertige Tablettenpalette.

### Untersuchung der ersten Tablettenformulierung

Zielsetzung ist es, pro Stamm $2*10^9$ KBE in einer Tablette a 400 mg zu verpressen.

*Tabelle 16: Rezeptur für 1 kg Pulvermischung* (+ *2,5* % *Palmfett als Trennmittel)*

| **Rezeptur für 1 kg** | |
|---|---|
| **Stoff** | **Menge [g]** |
| Topinambur | 332,500 |
| Agaveninulin | 332,500 |
| Baobab | 332,500 |
| L. plantarum | $4,125*10^{-2}$ |
| L. acidophilus | 0,100 |
| L. paracasei | $4,125*10^{-2}$ |
| B. longum | 0,713 |
| B. lactis | 1,663 |

*Bruchfestigkeit*

Ein wichtiger Parameter zur Bewertung der Tabletteneigenschaften ist die Bruchfestigkeit. Ist diese zu niedrig, zerfällt die Tablette möglicherweise schon im Mund, während eine zu harte Tablette den gesamten Darm passieren kann, ohne ihre Inhaltsstoffe freizusetzen.

Daher wurden drei Chargen der ersten Testpressung genauer untersucht. Charge 3 wurde hierbei noch ohne Palmfett als Trennmittel verpresst, Charge 4 und Charge 5 bereits mit. Weiterhin wurden drei verschiedene Prüfkräfte verwendet. Bei Charge 3 betrug die Kraft 2,15 kP (Gewichtskraft eines Kilogramms am Normort), bei Charge 4 schon 4,49 kP und bei Charge 5 schließlich 6,69 kP, um die Tablette zu brechen.

In Tabelle 17 ist klar ersichtlich, dass die steigende Presskraft mit einer zunehmenden Bruchfestigkeit in erwartungsgemäßer Art korreliert. Je höher die Presskraft, desto höher die Bruchfestigkeit. Ebenso lassen die im Rahmen der Messgenauigkeiten konstanten Bruchfestigkeiten auf eine gelungene Pressung schließen.

*Tabelle 17: Bruchfestigkeiten der unterschiedlichen Chargen der ersten Pressung*

| **Charge 3** | **Charge 4** | **Charge 5** |
|---|---|---|
| **Bruchfestigkeit [N]** | **Bruchfestigkeit [N]** | **Bruchfestigkeit [N]** |
| 88,20 | 72,20 | 110,60 |
| 89,00 | 80,20 | 134,40 |
| 83,20 | 110,80 | 145,20 |
| 66,00 | 74,40 | 131,00 |
| 79,20 | 102,60 | 105,60 |
| 58,80 | 86,00 | 124,20 |
| 68,20 | 76,40 | 124,20 |
| 68,60 | 88,00 | 105,40 |
| 69,80 | 90,20 | 142,20 |
| 87,20 | 83,20 | 117,00 |
| **75,82 (Mittelwert)** | **86,40 (Mittelwert)** | **123,98 (Mittelwert)** |

Die nächste Pressung erfolgte unter einem Druck, so dass Tabletten, die bei einer Presskraft von 4,49 kP (Charge 4) brechen, entstehen, da dies zu einer durchschnittlichen Bruchfestigkeit führt, die zumeist Anwendung in der Praxis findet.

*Zerfallszeit*

Die Bestimmung der Zerfallszeit erfolgte mittels einer sog. Basket-Apparatur, in der Tabletten in einem mit Wasser (37 °C) gefüllten Gehäuse durch einen Motor ständig auf und ab bewegt werden, wodurch die Magenperistaltik simuliert werden soll. Dabei betrug die Frequenz des Bewegung 30 dpm (dips per minute), wobei eine Strecke von 55 mm zurückgelegt wurde.

Es zeigten sich unter Berücksichtigung erstaunlich lange Zerfallszeiten der Tabletten. Um dies weiter zu verdeutlichen, wurde ein Vergleich angestellt. Den verwendeten Tabletten wurde eine Standardrezeptur der Pharma-Branche gegenübergestellt (s. Tabelle 18).

*Tabelle 18: Tablettenformulierung mit in der Pharma-Branche gängigen Stoffen*

| **Stoff** | **Funktion** | **Menge [%]** |
|---|---|---|
| Lactose Monohydrat | Füllmittel | 65 |
| PVP 30 | Binder | 5 |
| Magnesiumstearat | Schmiermittel | 3 |
| Mikrokristalline Cellulose | Formbeständigkeit | 22 |
| Wasser | Granulierung (trocken) | 5 |
| Pigment | Kontrolle | 0,025 |

Trotz einer relativ hohen Binderkonzentration (PVP 5 %) und einem Granulierschritt, wies Charge 4 des Synbiotikums, diejenige Charge, die auch für die folgenden Versuche von größter Relevanz ist, eine knapp drei Mal so hohe Zerfallszeit auf, wie die des vergleichbaren Pharmazeutischen Standards. Dies ist folgenden Tabellen (19 und 20) zu entnehmen.

*Tabelle 19: Gegenüberstellung der Bruchfestigkeit und der Zerfallszeit des pharm. Standards in Wasser*

| **Pharmazeutischer Standard** | | |
|---|---|---|
| **Charge** | **Br. Fest. [N]** | **Zerfallszeit (ZZ)** |
| **1** | 71,35 | 8 min 35 s |
| **2** | 92,68 | 9 min 56 s |
| **3** | 129,78 | 14 min 58 s |

*Tabelle 20: Gegenüberstellung der Bruchfestigkeit und der Zerfallszeit des Synbiotikums in Wasser und Magenmilieu (pH 1,5).*

| **Synbiotikum ZZ in Wasser** | | | **Synbiotikum ZZ bei pH = 1,5** | | |
|---|---|---|---|---|---|
| **Charge** | **Br. Fest. [N]** | **ZZ pH = 1,5** | **Charge** | **Br. Fest. [N]** | **ZZ Wasser** |
| **3** | 75,82 | 28 min 22 s | **3** | 75,82 | 32 min 30 s |
| **4** | 86,40 | 26 min 17 s | **4** | 86,40 | 29 min 20 s |
| **5** | 123,98 | 30 min 46 s | **5** | 123,98 | 33 min 35 s |

Demnach bildet die Zusammensetzung des Synbiotikums eine gute Grundvoraussetzung, auf der weiter aufgebaut werden kann, um die Magenpassage bestmöglich zu überstehen. Im simulierten Magenmilieu ist eine bis zu knapp 14%-ige Abnahme der Zerfallszeit zu beobachten. Dies kommt aufgrund des fehlenden Schutzes zustande. Die Säure kann die Tablette ungehindert angreifen.

**Untersuchung der zweiten Tablettenformulierung**

Aufgrund der guten Ergebnisse der Apfelfaser wurde diese als Hauptkomponente zur zweiten Rezeptur hinzugefügt. Außerdem wurden zwei Gelbildner untersucht. Zudem wurde Inulin ab der zweiten Charge weggelassen. Jedoch hatte sich herausgestellt, dass Inulin als Binder fungiert, weshalb es wichtig für die Festigkeit der Tablette sein kann. Es wurde Inulin nachträglich ab Charge 2 zu 25 % hinzugegeben. Tabelle 21 zeigt die Zusammensetzung der 6 verpressten Chargen (ohne zugegebenes Inulin).

*Tabelle 21: Rezeptur für 1 kg Pulvermischung (MO: Mikroorganismen; K-GM: Konjak Glucomannan; Angaben in %)*

| **Stoffe** | **Charge 1** | **Charge 2** | **Charge 3** | **Charge 4** | **Charge 5** | **Charge 6** |
|---|---|---|---|---|---|---|
| **Topinambur** | 30,0 | 30,0 | 30,0 | 30,0 | 40,0 | 40,0 |
| **Apfslfaser** | 40,0 | 40,0 | 40,0 | 40,0 | 40,0 | 40,0 |
| **Baobab** | 20,0 | 20,0 | 17,5 | 15,0 | 5,0 | 2,5 |
| **K-GM** | 5,0 | 7,5 | 10,0 | 12,5 | 10,0 | 12,5 |
| **Inulin** | 2,5 | | | | | |
| **Palmfett** | 2,5 | 2,5 | 2,5 | 2,5 | 2,5 | 2,5 |
| **Alginat** | | | | | 2,5 | 2,5 |
| **Summe** | 100,0 | 100,0 | 100,0 | 100,0 | 100,0 | 100,0 |

*Tabelle 22: Rezeptur für die zugesetzten Probiotika in 1 kg Pulvermischung*

| **Probiotika** | **In 1 kg Rezeptur [g]** |
|---|---|
| *L. plantarum* | 0,8 |
| *L. acidophilus* | 0,8 |

(fortgesetzt)

| Probiotika | In 1 kg Rezeptur [g] |
|---|---|
| *L. paracasei* | 0,8 |
| *B. lactis* | 0,3 |
| *B. longun,* | 0,3 |

*Bruchfestigkeit und Zerfallszeit*

Wie zuvor für die erste Tablettenformulierung beschrieben wurden auch die Tabletten der neuen Pressung analysiert. Von großem Interesse war dabei die hohe Zerfallszeit, die sich ergab (Tabelle 23). Obwohl durch die Apfelfaser ein enorm hoher Anteil an Sprengmittel in der Formulierung enthalten war, hielten sich die Tabletten zwischen 22 min 59 s und 29 min 37 s (Wasser) in der Basket-Apparatur. Die Tatsache, dass in der Apfelfaser natürlich enthaltene Pektin würde die Zerfallszeit positiv beeinflussen, konnte die Wirkung der Faser als Sprengmittel nicht kompensieren.

Eine Sprengmittelzugabe von 2 % reicht im Allgemeinen schon aus, um den Zerfall einer Tablette maßgeblich negativ zu beeinflussen. In der hier verwendeten Rezeptur fanden bis zu 40 % des Sprengmittels Anwendung, was für eine sehr gute Leistung der Gelbildner spricht.

*Tabelle 23: Gegenüberstellung der Bruchfestigkeit und der Zerfallszeit (ZZ) des Synbiotikums in Wasser und in simuliertem Magen- (pH = 1,5; Tabelle 31) sowie Darmmilieu (pH = 8,0)*

| **Synbiotikum** | | | | |
|---|---|---|---|---|
| **Charge** | **Br. Fest. [N]** | **ZZ Wasser** | **ZZ pH = 1,5** | **ZZ pH = 8,0** |
| **2** | 17,25 | 22 min 59 s | 22 min 08 s | 22 min 25 s |
| **3** | 18,66 | 26 min 22 s | 25 min 17 s | 26 min 36 s |
| **4** | 20,63 | 28 min 28 s | 28 min 03 s | 28 min 15 s |
| **5** | 19,14 | 23 min 05 s | 22 min 31 s | 22 min 48 s |
| **6** | 20,48 | 29 min 37 s | 29 min 16 s | 29 min 31 s |

*Ergebnis:* Beim nächsten Pressversuch sollte berücksichtigt werden, dass Inulin für die Festigkeit der aus der Rezeptur resultierenden Tabletten vorteilhaft und Apfelfaser nachteilig ist.

**Untersuchung der dritten Tablettenformulierung**

Neben den schon verwendeten Verdickungsmitteln bzw. Gelbildnern wurde hier auch Chia-Mehl und Agar-Agar näher untersucht. Ebenso wurde die zu Beginn hergenommene Rezeptur der ersten Pressung erneut verpresst, um weitere Rückschlüsse über die Lagerbedingungen zu ermöglichen.

*Tabelle 24: Rezeptur für Pulvermischung anteilig (MO: Mikroorganismen; K-GM: Konjak-Glucomannan;; Rez 1-10 = Charge 1-10)*

| **Stotte** | **Rez 1** | **Rez 2** | **Rez 3** | **Rez 4** | **Rez 5** | **Rez 6** | **Rez 7** | **Rez 8** | **Rez 9** | **Rez 10** |
|---|---|---|---|---|---|---|---|---|---|---|
| **Topinambur** | 5,0 | 21 | 21 | 22,5 | 20 | 31,5 | 31,5 | 37 | 26,0 | 33,0 |
| **Baobab** | 10,0 | 13,0 | 3,0 | 22,5 | 20 | 22,5 | 12,5 | 27,0 | 27,0 | 33,3 |
| **K-GM** | 10,0 | 1,0 | 1,0 | 1 | - | 1,0 | 1,0 | 1,0 | 18,0 | |
| **Inulin** | 39,7 | 39,7 | 39,7 | 22,7 | 29,7 | 39,7 | 39,7 | 19,7 | 28,7 | 33,3 |
| **Probiotika** | 0,3 | 0,3 | 0,3 | 0,3 | 0,3 | 0,3 | 0,3 | 0,3 | 0,3 | 0,3 |
| **Agar-Agar** | - | 0 | 0 | 25 | 15 | 10 | 10 | 10 | - | - |
| **Alginat** | 5,0 | 5,0 | 5,0 | - | - | 5,0 | 5,0 | 5,0 | - | - |
| **Chia Mehl** | 30,0 | 20,0 | 30,0 | - | - | - | - | - | - | - |

(fortgesetzt)

| Stotte | Rez 1 | Rez 2 | Rez 3 | Rez 4 | Rez 5 | Rez 6 | Rez 7 | Rez 8 | Rez 9 | Rez 10 |
|---|---|---|---|---|---|---|---|---|---|---|
| **Palmfett** | 2,5 | 2,5 | 2,5 | - | - | 2,5 | 2,5 | 2,5 | 2,5 | 2,5 |
| **Reisextrakt** | - | - | - | 2 | 5 | - | - | - | - | - |
| **Maissiruppulver** | - | - | - | 3 | 7 | - | - | - | - | - |
| **Silizium Dioxid** | - | - | - | 1 | 3 | - | - | - | - | - |
| **Summe** | 102,5 | 102,5 | 102,5 | 100,0 | 100,0 | 102,5 | 102,5 | 102,5 | 102,5 | 102,5 |

*Tabelle 25: Rezeptur für die zugesetzten Probiotika in 1 kg Pulvermischung*

| Probiotika | In 1kg Rezeptur [g] |
|---|---|
| *L. plantarum* | 0,8 |
| *L. acidophilus* | 0,8 |
| *L. paracasei* | 0,8 |
| *B. lactis* | 0,3 |
| *B. longum* | 0,3 |

*Bruchfestigkeit und Zerfallszeit*

In Tabelle 26 sind die Zerfallszeiten der dritten Pressung aufgelistet. Charge 1 bis 3 enthielten unterschiedliche Konzentrationen von Chia-Mehl. Die Zerfallszeiten waren mit einer knappen halben Stunde recht gut. Allerdings besitzt die Schale der Chiasamen scheinbar auch einen elastischen Anteil, der zu einer niedrigeren Bruchfestigkeit geführt haben könnte.

Charge 4 und 5 waren überraschenderweise die vielversprechendsten. Bei diesen waren Agar-Agar zugesetzt, wodurch sich ein Gel um die Tablette bildete, die den Zerfall verzögerte, sowie Kombinationen der Hilfsstoffe Reisextrakt, Maissiruppulver und Siliciumdioxid. Charge 9 war an die Rezeptur der ersten Verpressung angelehnt, also Baobab, Topinambur und Inulin zu gleichen Anteilen, dieses Mal jedoch mit dem Zusatz von Gelbildnern. Dabei erreichte die Formulierung eine Zerfallszeit im Sauren von über 49 min. Damit ist dies die dritthöchste Zerfallszeit. Hier wurden höhere Anteile an Konjak-Glucomannan verwendet. Konjak-Glucomannan hat jedoch den Nachteil, dass es ein sehr großes Quellvermögen aufweist und insofern in hohen Anteilen beim Verzehr nicht geeignet sein könnte (Steckenbleiben in Speiseröhre).

Charge 10 war die wiederholte Verpressung der ersten Rezeptur. Die Bruchfestigkeit hierbei war deutlich niedriger, als sie es bei den gelagerten Tabletten war. Da die Parameter der Pressung beinahe identisch waren und dieselben Stoffe verwendet wurden, deutet dies auf eine Nachhärtung der Tabletten hin. Das ist kein Sonderfall. Durch das stark hygroskopische Inulin, wird viel Wasser gebunden, das wiederum über eine nicht geschlossene Umgebung zu eben dieser Nachhärtung führt. Diese Nachhärtung kann durch eine Lagerung in einem geeigneten Packungsmaterial vermieden werden.

*Tabelle 26: Gegenüberstellung der Bruchfestigkeit und der Zerfallszeit (ZZ) des Synbiotikums in Wasser und in simuliertem Magen- (pH = 1,5) sowie Darmmilieu (pH = 8,0) (Prüfkraft bei Tablettenbruch ca. 6 kP)*

| **Synbiotikum** | | | | |
|---|---|---|---|---|
| **Charge** | **Br. Fest. [N]** | **ZZ Wasser** | **ZZ pH = 1,5** | **ZZ pH = 8,0** |
| **1** | 19,56 | 33 min 17 s | 27 min 58 s | 32 min 54 s |
| **2** | 21,37 | 29 min 23 s | 26 min 04 s | 29 min 11 s |
| **3** | 22,49 | 28 min 41 s | 27 min 18 s | 28 min 25 s |
| **4** | 45,16 | 1 h 16 min | 1 h 01 min | 1 h 09 min |
| **5** | 48,34 | 1 h 14 min | 1 h 00 min | 1 h 06 min |

(fortgesetzt)

| **Synbiotikum** | | | | |
|---|---|---|---|---|
| **Charge** | **Br. Fest. [N]** | **ZZ Wasser** | **ZZ pH = 1,5** | **ZZ pH = 8,0** |
| **6** | 42,29 | 30 min 35 s | 28 min 15 s | 30 min 11 s |
| **7** | 46,87 | 29 min 45 s | 25 min 58 s | 29 min 40 s |
| **8** | 42,18 | 35 min 50 s | 32 min 45 s | 34 min 18 s |
| **9** | 44,64 | 53 min 24 s | 49 min 02 s | 52 min 54 s |
| **10** | 48,71 | 15 min 37 s | 13 min 12 s | 15 min 42 s |

In Figur 8 ist die Zerfallszeit im Magenmilieu bei unterschiedlichen Tablettenhärten über die verschiedenen Chargen hinweg aufgeführt. Hierbei zeigt sich ein weiteres Mal, dass die Formulierungen mit dem Chia-Mehl (Charge 1-3) aufgrund der Elastizität der Schalen zu relativ weichen, dafür aber recht resistenten Tabletten führen.

Charge 4 und 5 waren wie schon erwähnt die besten Rezepturen hinsichtlich der Magenverweilzeit. Der starke Abfall der Zerfallszeit bei unterschiedlichen Tablettenhärten bzw. verschiedenen Presskräften ist hier am deutlichsten. Bei niedrigeren Tablettenhärten läuft der Zerfall bis zu 13 Minuten schneller ab, was in etwa 20 % der gesamten Zerfallszeit entspricht.

Zudem zeigt sich in Figur 8 deutlich, welch starken Einfluss die Nachhärtung, oder im Allgemeinen die Tablettenhärte, auf die Zerfallszeit hat. Charge 10 enthält die gleichen Stoffe wie die Tabletten der erste Verpessung, zerfällt aber in der Hälfte der Zeit. Ebenso ist natürlich die Bruchfestigkeit der gelagerten Tabletten enorm gestiegen. Hier hat sich der Wert von 86,40 N zu 48,71 N beinahe verdoppelt.

Figur 9 stellt den direkten Masseverlust über die Zeit dar. Hier wurden jeweils 6 bis 8 Tabletten untersucht und zu unterschiedlichen Zeiten der Basket-Apparatur entnommen. Interessant zu beobachten ist hier die anfangs nur mäßige Masseabnahme.

Interessant zu beobachten ist hier die anfangs nur mäßige Masseabnahme. Dies kommt womöglich durch die Wasseraufnahme der Gelbildner zustande, was zu einer Stabilisierung der Tablette führt.

Auch hier schneiden die Formulierungen mit hohen Anteilen an Agar-Agar, Glucomannan und den Hilfsstoffen Reisextrakt, Maissiruppulver und Siliciumdioxid am besten ab. Deren Zerfallszeit betrug jeweils ca. eine Stunde (Charge 4 und 5 und 9).

*Einfluss der Pressung auf die Viabilität*

In der Regel führt ein höherer Pressdruck zu einem erhöhten Absterben der Keime. Es wurden pro Charge Tabletten mit einem Pressdruck von 4,5 kP und 6 kP hergestellt. Zu erwarten wäre eine höhere Viabilität bei den Tabletten mit der geringeren Härte. Die Unterschiede zwischen den Keimzahlen der Tabletten, die bei 4,5 kP hergestellt wurden, und den Tabletten, die bei 6 kP hergestellt wurden, war so gering, dass es nicht möglich war, die genauen Abweichungen mit Hilfe der verwendeten mikrobiologischen Analysen deutlich zu machen. Tabelle 27 zeigt, dass bei einem Pressdruck von 6 kP ca. 25-50 % der Keime abgestorben sind. Dies zeigt, dass nach dem Pressen noch genug lebende Keime in der Tablette vorzufinden, was der Fall war.

*Tabelle 27: Bestimmung der Keimzahl zweier Chargen direkt nach der Pressung (Härte: 6 kP)*

| | **-10** | | | | **-11** | | | | **-12** | | | | **KBE** |
|---|---|---|---|---|---|---|---|---|---|---|---|---|---|
| **Charge 4** | 182 | 385 | 432 | 314 | 40 | 15 | 18 | 32 | 14 | 4 | 7 | 11 | |
| Mittelwert | 328,25 | | | | 26,25 | | | | 9 | | | | **$5{,}0*10^{16}$** |
| **Charge 5** | 128 | 850 | 750 | 850 | 26 | 72 | 69 | 89 | 8 | 9 | 16 | 7 | |
| Mittelwert | 644,5 | | | | 64 | | | | 10 | | | | **$7{,}6*10^{16}$** |

**8. Untersuchung der Lagerfähigkeit**

Neben der Magenpassage ist die genauere Untersuchung der bei der Lagerung auftretenden Veränderungen hinsichtlich der Viabilität der Mikroorganismen in einer Tablette von großem Interesse. Es wurde ein Lagerversuch bei Raumtemperatur über 12 Monate sowie ein Stresstest bei 40 °C über 3 Monate durchgeführt. Hierbei wurden jeweils die Tabletten des ersten Pressversuchs verwendet.

*Durchführung*

Es wurde jeweils eine Tablette (400 mg) in 40 ml PBS gelöst. Dazu wurde die Tablette plus Lösungsmittel in einem Sterilgefäß auf eine Schüttelplatte gegeben und bei 200 rpm (rotations per minute) geschüttelt. Die Zeit bis zur Auflösung der Tablette betrug ca. zweieinhalb Stunden. Anschließend wurden jeweils 100 μl der Proben genommen und in das erste Eppendorf-Reaktionsgefäß der Verdünnungsreihe gegeben und stellten somit die stock solution. Diese wurde in 1:10 Schritten verdünnt und ausplattiert. Das Vorgehen war hier analog dem der Einzelkulturansiedlung.

*Homogenität*

Da hier zahlreiche Tabletten gelöst und Proben davon ausplattiert wurden, bot sich dieser Versuch ebenso zur Untersuchung der Homogenität der Keimzahlverteilung an. Es wurden hierfür insgesamt zwanzig Tabletten pro Charge 4 und 5 der ersten Pressung *(s. Untersuchung der ersten Tablettenformulierung)* sowie zehn Tabletten der Chargen 4 und 5 der dritten Pressung *(s. Untersuchung der ersten Tablettenformulierung)* gelöst und ausplattiert. Bei den Chargen der ersten Pressung konnten Keimzahlen zwischen $1*10^{14}$ und $9,5*10^{14}$ beobachtet werden. Tabelle 29 zeigt hier einen Ausschnitt der ermittelten Werte. Bei den Chargen der dritten Pressung wurden Keimzahlen zwischen $2*10^{16}$ und $8*10^{16}$ ermittelt. Ebenso zeigt hier Tabelle 27 einen Ausschnitt aus den Messwerten.

**Lagerung bei Raumtemperatur**

In Tabelle 28 sind in der zweiten Spalte die vom Hersteller (IP Ingredients) angegebenen KBE/g nach Ablauf des MHD (12 Monate) angegeben. In Spalte drei sind die mittels mikrobiologischer Verfahren gewonnenen wirklichen KBE/g zum Zeitpunkt der Versuche aufgeführt. Spalte vier enthält die in die Rezepturen der ersten Verpressung zugegebenen Mengenangaben. Daraus ließ sich, wie in Spalte fünf gezeigt, die Gesamtkeimzahl ($1,63*10^{16}$) einer Tablette bestimmen.

*Tabelle 28: Keimberechnung der in der ersten Verpressung enthaltenen Keime (Anzahl/g (Mibi) stellt hierbei die Referenz zum Zeitpunkt t=0 für meine Versuche dar)*

| | **Anzahl/g (MHD)** | **Anzahl/g (Mibi)** | **In Rezeptur [g]** | **Anzahl/Tablette** |
|---|---|---|---|---|
| **L. plantarum** | $4*10^{11}$ | **$4,77*10^{16}$** | 0,04125 | $1,97*10^{15}$ |
| **L. acidophilus** | $2*10^{11}$ | **$5,96*10^{16}$** | 0,1 | $5,96*10^{15}$ |
| **L. paracasei** | $4*10^{11}$ | **$2,13*10^{16}$** | 0,04125 | $8,79*10^{14}$ |
| **B. longum** | $1*10^{11}$ | **$2,59*10^{15}$** | 0,7125 | $1,85*10^{15}$ |
| **B. lactis** | $5*10^{11}$ | **$3,38*10^{15}$** | 1,6625 | $5,62*10^{15}$ |
| **Summe** | - | **-** | - | **$1,63*10^{16}$** |

Um das Absterben der Probiotika nun genauer evaluieren zu können, muss ein Vergleich zwischen den KBE nach der jeweiligen Lagerdauer und den KBE zu Beginn gezogen werden. Hierbei ist ersichtlich, dass sowohl bei Charge 4 als auch bei Charge 5 (Tabelle 29) die Keimzahl um ca. zwei Zehnerpotenzen gesunken ist. Weiterhin ist im Rahmen der mikrobiologischen Messgenauigkeiten festzustellen, dass die bei Charge 5 angewandte, höhere Presskraft keinen sichtbaren Einfluss auf das Überleben der Keime über eine Lagerdauer von 12 Monaten hinweg mit sich bringt.

*Tabelle 29: Keimzahl der Mikroorganismen in den Tabletten (Charge 4: C4; Charge 5: C5; Verdünnungsstufe $10^{-8}$ und $10^{-9}$) nach 12 Monaten bei Raumtemperatur*

| | -8 | | | | -9 | | | | KBE |
|---|---|---|---|---|---|---|---|---|---|
| **Lager C4** | 630 | 640 | 524 | 609 | 84 | 72 | 69 | 71 | |
| Mittelwert | 600,75 | | | | 74 | | | | **6,70*10^14** |

| | | | | | | | | | |
|---|---|---|---|---|---|---|---|---|---|
| **Lager C5** | 130 | 215 | 286 | 195 | 14 | 16 | 25 | 32 | |
| Mittelwert | 206,5 | | | | 21,75 | | | | **2,12*10^14** |

**Stresstest**

Die in folgender Tabelle aufgeführten Werte vertreten die mikrobiologischen Analysen der unter Stressbedingungen gelagerten Tabletten. Hierbei ist klar ersichtlich, dass sich der Stresstest nicht negativ auswirkt im Gegensatz zu der Lagerung unter Normalbedingungen. Dies liegt vermutlich daran, dass die Milchsäurebakterien sich gerade bei einer Temperatur um die 40 °C sehr wohl fühlen und demnach die Zahl der KBE in vergleichbarem Maß wie bei Raumtemperatur sinkt.

Generell ist nach über eine Lagerdauer eine Senkung um ebenso zwei Zehnerpotenzen im Bereich unserer Erwartungen. Um diesem Absterben entgegen zu wirken, wurden in die Tabletten des dritten Pressversuchs weitaus mehr Probiotika hinzugegeben. Damit ist sichergestellt, dass die Tabletten am Ende einer Lagerperiode von 12 Monaten bei Raumtemperatur oder 3 Monaten bei 40 °C noch so viele Keime enthalten, um im Darm mit einer Konzentration von mindestens $1*10^{10}$ Keimen anzukommen.

*Tabelle 30: Keimzahl der Mikroorganismen in den Tabletten (Charge 4: C4; Charge 5: C5; Verdünnungsstufe $10^{-8}$ und $10^{-9}$) nach 3 Monaten bei 40 °C*

| | -8 | | | | -9 | | | | KBE |
|---|---|---|---|---|---|---|---|---|---|
| **Stresstest C4** | 650 | 700 | 400 | 550 | 66 | 52 | 71 | 24 | |
| Mittelwert | 575 | | | | 53,25 | | | | **5,54*10^14** |
| **Stresstest C5** | 850 | 900 | 450 | 600 | 111 | 69 | 84 | 15 | |
| Mittelwert | 700 | | | | 69,75 | | | | **6,99*10^14** |

## 9. Vergleich unterschiedlicher Formulierungen

Die Formulierung in Tablettenform soll besondere Vorteile gegenüber anderen Formulierungen mit sich bringen. Im Vergleich zur Kapsel können in einer Tablette weit mehr Inhaltsstoffe untergebracht werden. Hinsichtlich einer Pulverformulierung sollte die Tablette einen weitaus besseren Säureschutz gewährleisten. Im folgenden Versuch wurde ein Vergleich der Absterberate über die Zeit zwischen verschiedenen Formulierungen gezogen.

*Durchführung*

Es wurden vier Sterilgefäße mit 50 ml eines simulierten Magensaftes (Tabelle 31) befüllt. In zwei wurden zehn Tabletten der Charge 4 bzw. Charge 5 hinzugegeben, in eines wurde die Pulvermischung für zehn Tabletten gegeben und in das dritte Gefäß wurden ausschließlich 12 mg der Mischkultur als Referenz hinzugefügt. Da in einer Tablette 0,12 mg der Mischkultur enthalten sind, entsprach dies dem hundertfachen Wert. Die Schüttelplatte war hierbei auf 150 rpm eingestellt. Nun wurde alle zwanzig Minuten eine 100 µl-Probe aus jedem Gefäß genommen, entsprechend verdünnt und anschließend ausplattiert.

*Tabelle 31: Zusammensetzung des synthetischen Magensaftes*

| Synthetischer Magensaft (100 ml) | |
|---|---|
| NaCl | 290 mg |
| $KH_2PO_4$ | 27 mg |
| Pepsin | 100 mg |
| KCl | 70 mg |
| HCl | (Einstellung auf pH 1,5-2) |

*Ergebnisse*

Wie erhofft zeigt die Tablettenformulierung in Figur 10 einen deutlich erkennbaren Schutz gegenüber dem Magensaft. Während die Keimzahl der Referenzprobe dem sauren Milieu über zwei Stunden hinweg sehr stark nachgab (von 1 *$10^{17}$ KBE auf 1,3*$10^8$ KBE), hielt sich das Pulver teilweise ein klein wenig besser.

Die Keimzahlen der Charge 4 und Charge 5 sanken in etwa gleichem Maß, jedoch klar langsamer als die der Referenz und des Pulvers. Zu Beginn (t = 20-60 min) war kaum eine Abnahme zu erkennen, da die Tablette noch fast ganz war und die starke Wasseraufnahme durch die Gelbildner scheinbar sehr gut vor der Säure schützte. Je mehr die Tablette zerfällt, umso mehr Keime sterben dann ab. Dennoch ist die Überlebensrate der Keime in einem sehr guten Rahmen. So überleben nach zwei Stunden etwa $10^{12}$ bis $10^{13}$ Keime der Tablettenproben.

**Patentansprüche**

1. Stoffzusammensetzung zum Aufbau einer gesundheitsfördernden Darmflora, **dadurch gekennzeichnet, dass** sie

   - zwei oder mehr Präbiotika und
   - Probiotika umfassend Lactobacillus plantarum, Lactobacillus acidophilus, Lactobacillus paracasei, Bifidobacterium lactis und Bifidobacterium longum umfasst.

2. Stoffzusammensetzung gemäß Anspruch 1, **dadurch gekennzeichnet, dass** die Stoffzusammensetzung als Tablette oder Kapsel vorliegt.

3. Stoffzusammensetzung gemäß eines der vorherigen Ansprüche, **dadurch gekennzeichnet, dass** 75-99,7 Gew.-% der Stoffzusammensetzung aus Präbiotika bestehen.

4. Stoffzusammensetzung gemäß eines der vorherigen Ansprüche, **dadurch gekennzeichnet, dass** die Stoffzusammensetzung pro Gramm 1x$10^8$- 1x$10^{17}$, bevorzugt 1x$10^{10}$ - 1x$10^{14}$, besonders bevorzugt 1x$10^{11}$ - 1x$10^{13}$ Koloniebildende Einheiten (KBE) der Probiotika enthält.

5. Stoffzusammensetzung gemäß eines der vorherigen Ansprüche, **dadurch gekennzeichnet, dass** die Probiotika rechtsdrehende Milchsäure produzieren.

6. Stoffzusammensetzung gemäß eines der vorherigen Ansprüche, **dadurch gekennzeichnet, dass** die Stoffzusammensetzung mindestens ein Präbiotikum ausgewählt aus der Gruppe umfassend Topinambur, Baobab, Inulin, Konjakwurzel, Alginat, Agar-Agar, Guarkernmehl und/oder Xanthan enthält.

7. Stoffzusammensetzung gemäß eines der vorherigen Ansprüche, **dadurch gekennzeichnet, dass** die Stoffzusammensetzung aus Bestandteilen in Bio-Qualität gemäß EG-Öko-Verordnung Nr. 834/2007 besteht, in ihrer Gesamtheit Bio-Qualität gemäß EG-Öko-Verordnung Nr. 834/2007 aufweist und/oder in ihrer Gesamtheit Bio-Qualität gemäß den Richtlinien des United States of America National Organic Program (USDA Organic) aufweist.

8. Stoffzusammensetzung gemäß eines der vorherigen Ansprüche, **dadurch gekennzeichnet, dass** die Stoffzusammensetzung keine tierischen Bestandteile enthält.

9. Stoffzusammensetzung gemäß eines der vorherigen Ansprüche, **dadurch gekennzeichnet, dass** die Stoffzusammensetzung in Form einer Tablette mit einer Bruchfestigkeit von mindestens 16 Newton vorliegt.

## Claims

1. A material composition to build up a health promoting intestinal flora, **characterized in that** it comprises

   - two or more prebiotics, and
   - probiotics comprising Lactobacillus plantarum, Lactobacillus acidophilus, Lactobacillus paracasei, Bifidobacterium lactis and Bifidobacterium longum.

2. The material composition according to claim 1, **characterized in that** the material composition is in the form of a tablet or capsule.

3. The material composition according to any one of the preceding claims, **characterized in that** 75-99.7% by weight of the material composition consists of prebiotics.

4. The material composition according to any one of the previous claims, **characterized in that** the material composition contains $1x10^8$- $1x10^{17}$, preferably $1x10^{10}$ - $1x10^{14}$, particularly preferably $1x10^{11}$ - $1x10^{13}$ colony forming units (CFU) of the probiotics per gram.

5. The material composition according to any one of the preceding claims, **characterized in that** the probiotics produce clockwise rotating lactic acid.

6. The material composition according to any one of the preceding claims, **characterized in that** the material composition contains at least one prebiotic selected from the group comprising topinambour, baobab, inulin, konjac root, alginate, agar-agar, guar gum and/or xanthan.

7. The material composition according to any one of the preceding claims, **characterized in that** the material composition consists of constituents in bio quality according to EC Eco regulation no. 834/2007, in its entirety has bio quality according to EC Eco regulation no. 834/2007 and/or in its entirety has bio quality according to the policies of the United States of America National Organic Program (USDA Organic) .

8. The material composition according to any one of the preceding claims, **characterized in that** the material composition does not contain any animal components.

9. The material composition according to any one of the preceding claims, **characterized in that** the material composition is in the form of a tablet with a breaking strength of at least 16 Newtons.

## Revendications

1. Composition de matière destinée au développement d'un microbiote sain, **caractérisée en ce qu'**elle comprend

   - deux prébiotiques ou plus et
   - des probiotiques comprenant des Lactobacillus plantarum, des Lactobacillus acidophilus, des Lactobacillus paracasei, des Bifidobacterium lactis et des Bifidobacterium longum.

2. Composition de matière selon la revendication 1, **caractérisée en ce que** la composition de matière se présente sous forme de comprimés ou de capsules.

3. Composition de matière selon l'une des revendications précédentes, **caractérisée en ce que** 75 à 99,7 % en poids de la composition de matière consistent en des prébiotiques.

4. Composition de matière selon l'une des revendications précédentes, **caractérisée en ce que** la composition de matière contient, par gramme, de $1x10^8$ à $1x10^{17}$, de préférence de $1x10^{10}$ à $1x10^{14}$, de manière particulièrement préférée de $1x10^{11}$ à $1x10^{13}$ unités formatrices de colonies (KBE) des probiotiques

5. Composition de matière selon l'une des revendications précédentes, **caractérisée en ce que** les probiotiques produisent un acide lactique dextrogyre.

6. Composition de matière selon l'une des revendications précédentes, **caractérisée en ce que** la composition de matière contient au moins un prébiotique choisi dans le groupe comprenant le topinambour, le baobab, l'inuline, la racine de konjac, l'alginate, l'agar-agar, la farine de graine de guar et/ou le xanthane.

7. Composition de matière selon l'une des revendications précédentes, **caractérisée en ce que** la composition de matière est constituée de composants d'une qualité biologique conforme au Règlement ECO CE n° 834/2007, présente dans sa totalité une qualité biologique conforme au Règlement ECO CE n° 834/2007 et/ou présente dans sa totalité une qualité biologique conforme aux directives du National Organic Program des Etats-Unis d'Amérique (USDA Organic).

8. Composition de matière selon l'une des revendications précédentes, **caractérisée en ce que** la composition de matière ne contient pas de composant animal.

9. Composition de matière selon l'une des revendications précédentes, **caractérisée en ce que** la composition de matière se présente sous forme d'un comprimé avec une force de rupture d'au moins 16 newtons.

Figur 1

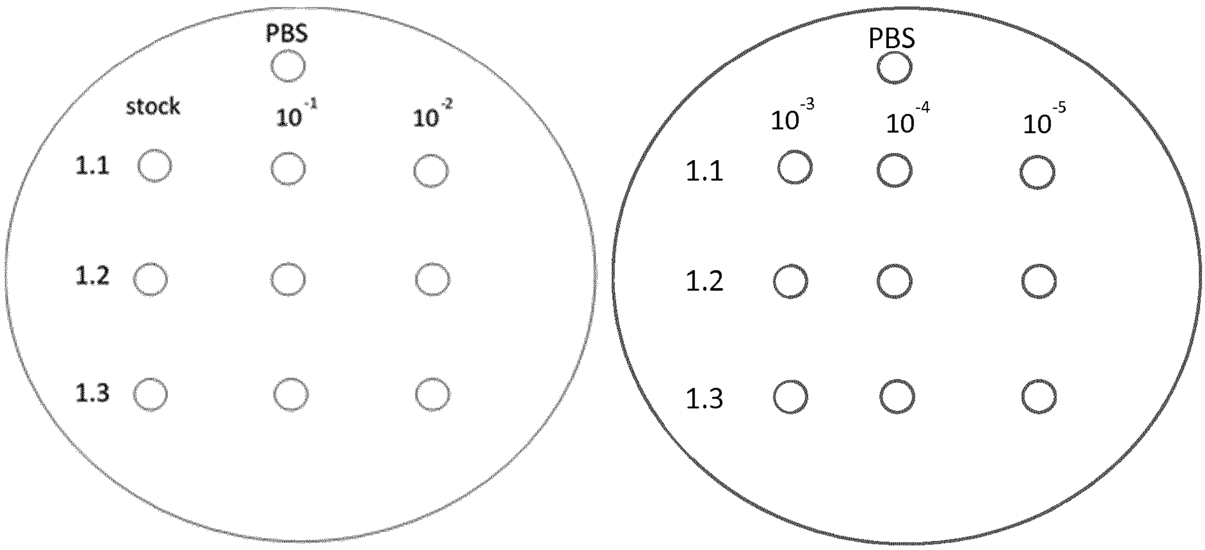


Figur 2

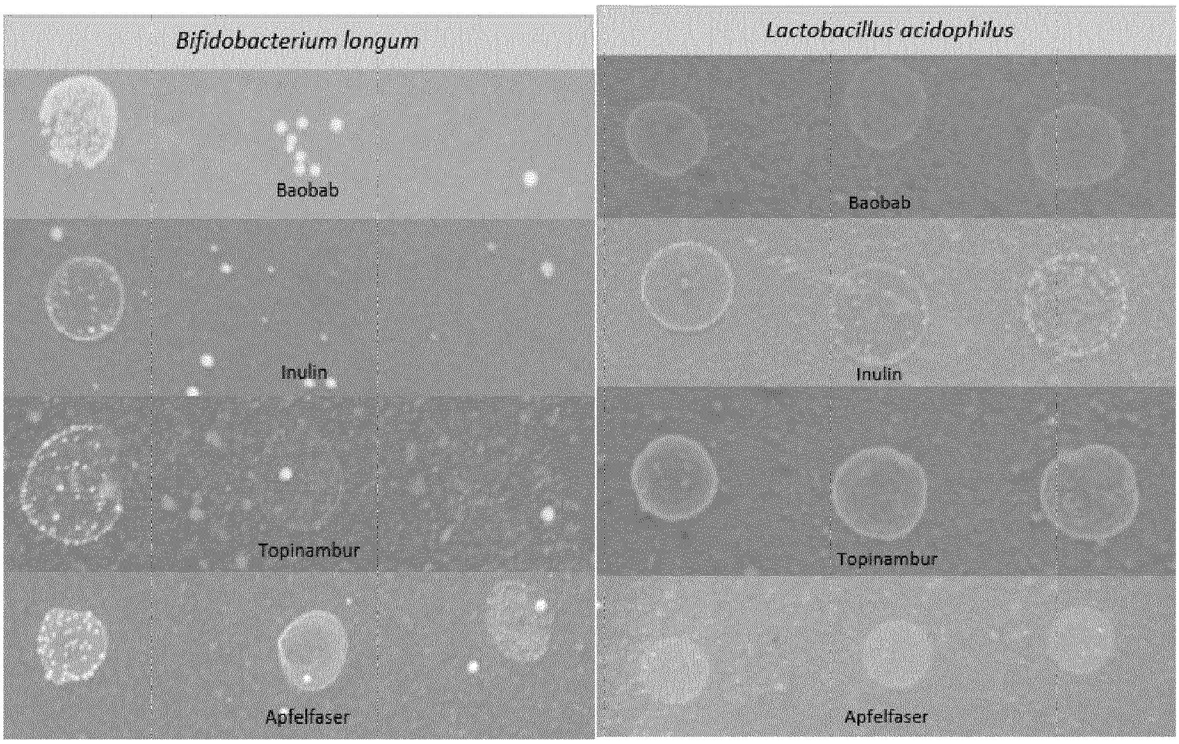

Figur 3

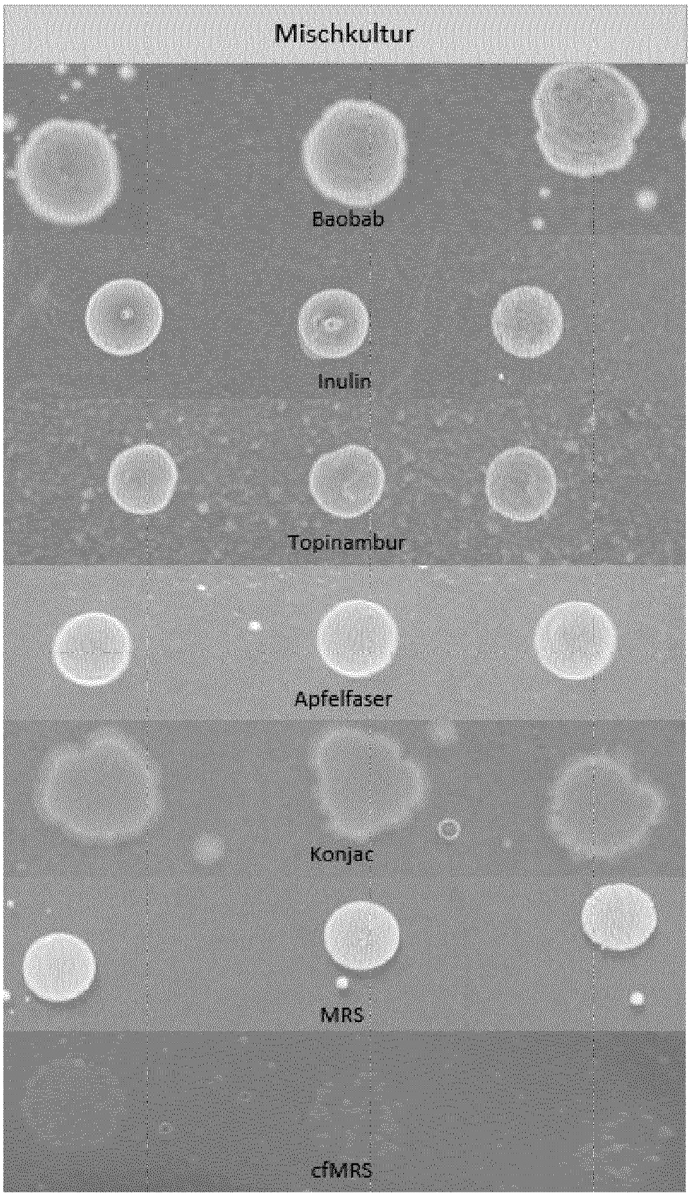


Figur 4

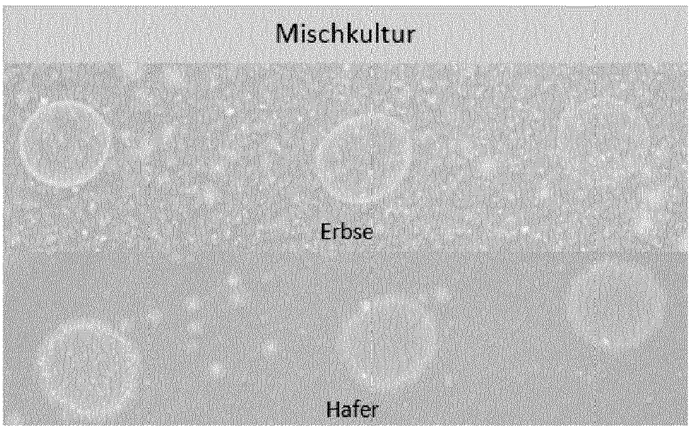

Figur 5

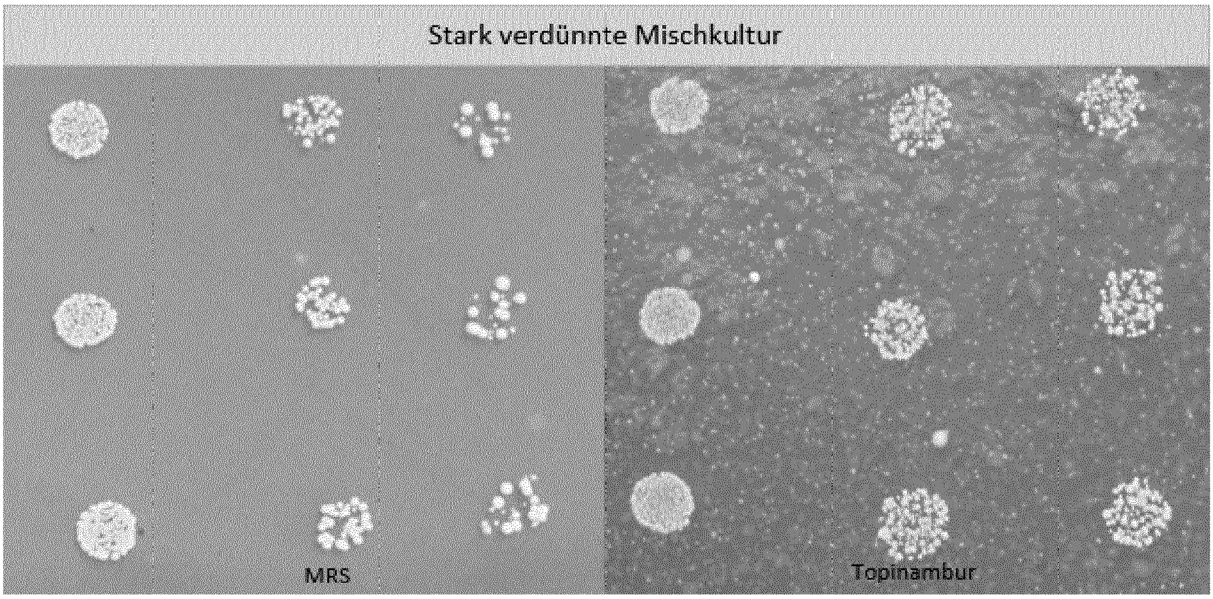


Figur 6

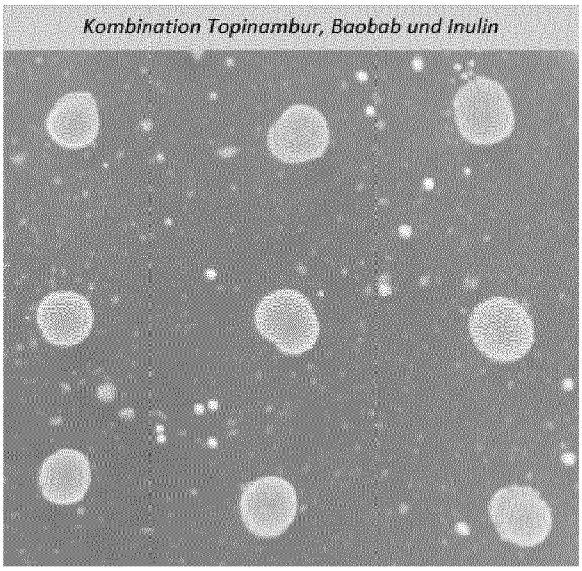

Figur 7

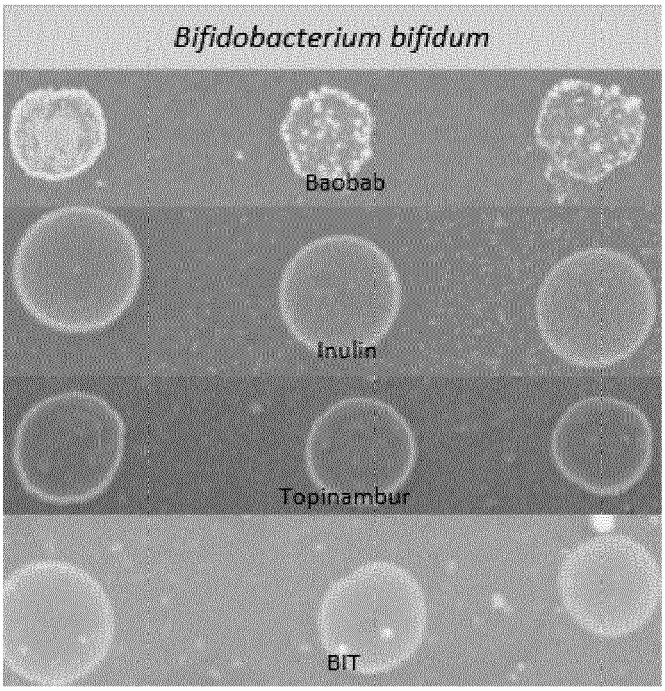


Figur 8

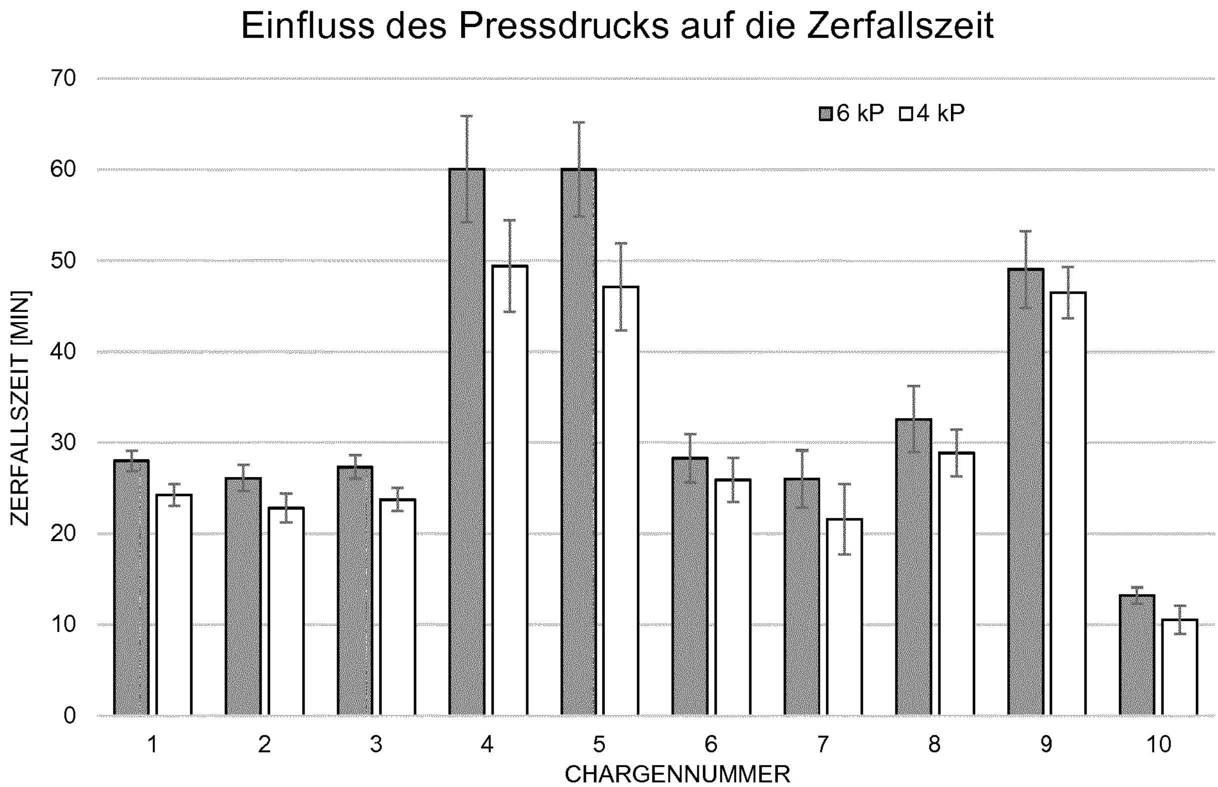

Figur 9

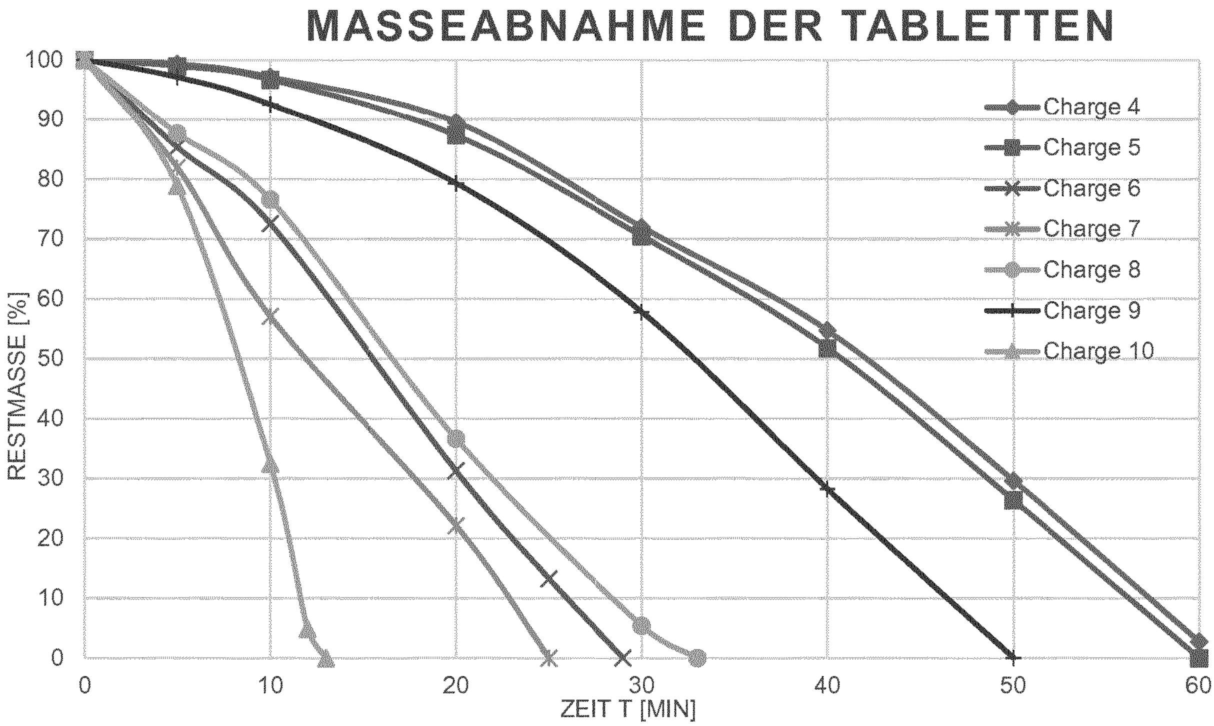
MASSEABNAHME DER TABLETTEN
100
90
80
70
60
50
40
30
20
10
0
RESTMASSE [%]
0
10
20
30
40
50
60
ZEIT T [MIN]
Charge 4
Charge 5
Charge 6
Charge 7
Charge 8
Charge 9
Charge 10

Figur 10

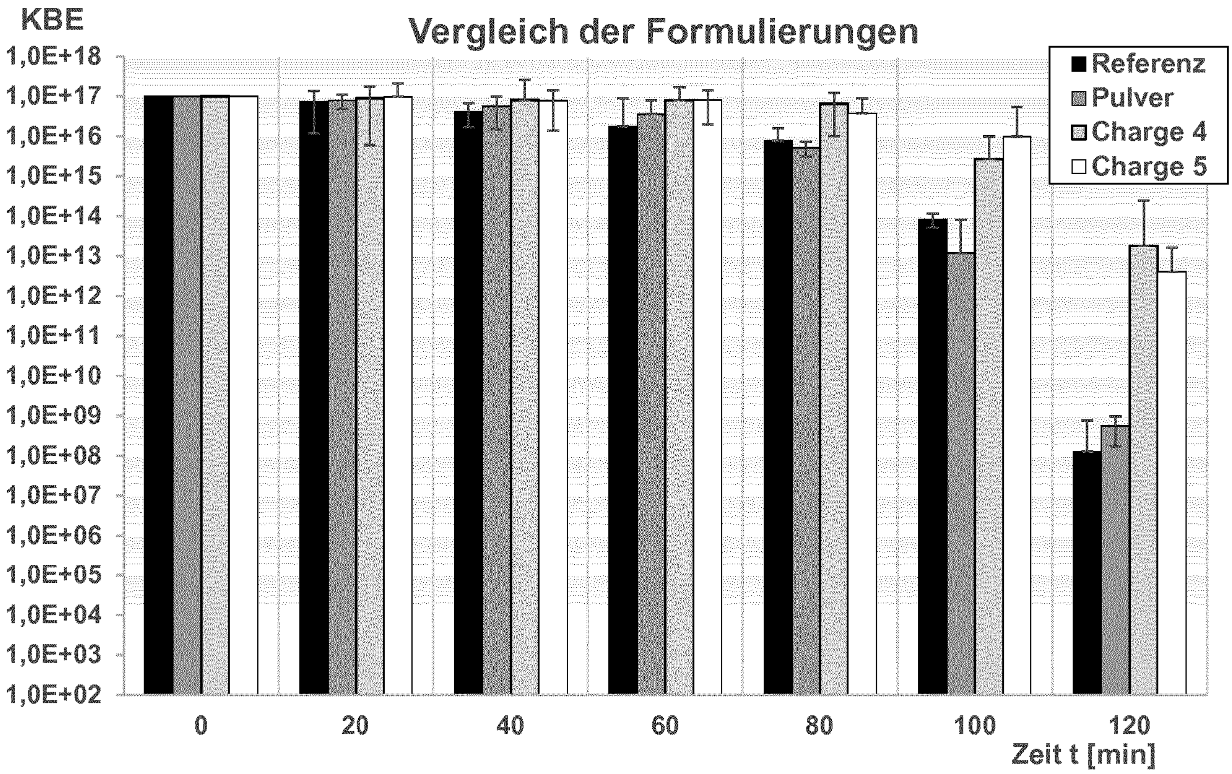
KBE
Vergleich der Formulierungen
1,0E+18
1,0E+17
1,0E+16
1,0E+15
1,0E+14
1,0E+13
1,0E+12
1,0E+11
1,0E+10
1,0E+09
1,0E+08
1,0E+07
1,0E+06
1,0E+05
1,0E+04
1,0E+03
1,0E+02
Referenz
Pulver
Charge 4
Charge 5
0
20
40
60
80
100
120
Zeit t [min]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 60020991 T2
- US 2008219961 A1
- US 20050220776 A1
- WO 2005056023 A1
- US 20170100442 A1


### In der Beschreibung aufgeführte Nicht-Patentliteratur


- *Carcinogenesis,* 1998, vol. 19, 281-285